(19) **Europäisches Patentamt / European Patent Office / Office européen des brevets**

(11) **EP 4 168 585 B1**

(12) # EUROPEAN PATENT SPECIFICATION

(45) Date of publication and mention of the grant of the patent:
**30.07.2025 Bulletin 2025/31**

(21) Application number: **21734113.0**

(22) Date of filing: **18.06.2021**

(51) International Patent Classification (IPC):
**C12Q 1/6883** (2018.01)

(52) Cooperative Patent Classification (CPC):
**C12Q 1/6883; C12Q 2600/158**

(86) International application number:
**PCT/EP2021/066586**

(87) International publication number:
**WO 2021/255237 (23.12.2021 Gazette 2021/51)**

(54) **METHOD OF DETECTING INFECTION WITH PATHOGENS CAUSING TUBERCULOSIS**

VERFAHREN ZUM NACHWEIS DER INFEKTION MIT TUBERKULOSEHERVORRUFENDEN PATHOGENEN

PROCÉDÉ DE DÉTECTION D'UNE INFECTION PAR DES PATHOGÈNES PROVOQUANT LA TUBERCULOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.06.2020 EP 20181107**

(43) Date of publication of application:
**26.04.2023 Bulletin 2023/17**

(73) Proprietor: **Mikrogen GmbH**
**82061 Neuried (DE)**

(72) Inventors:
- **DEML, Ludwig**
  **93128 Regenstauf (DE)**
- **BARABAS, Sascha**
  **93077 Bad Abbach (DE)**
- **RASCLE, Anne**
  **93080 Pentling (DE)**
- **MEIER, Johannes P.**
  **84307 Eggenfelden (DE)**
- **GULDAN, Harald**
  **93133 Burglengenfeld (DE)**
- **ASBACH-NITZSCHE, Alexandra**
  **93051 Regensburg (DE)**

(74) Representative: **Rückerl, Florian**
**Dehmel & Bettenhausen**
**Patentanwälte PartmbB**
**Herzogspitalstraße 11**
**80331 München (DE)**

(56) References cited:
**WO-A2-2020/127908     KR-A- 20120 103 387**

- **SUNGHYUN KIM ET AL: "Diagnostic Performance of a Cytokine and IFN-[gamma]-Induced Chemokine mRNA Assay after Mycobacterium tuberculosis-Specific Antigen Stimulation in Whole Blood from Infected Individuals", THE JOURNAL OF MOLECULAR DIAGNOSTICS, vol. 17, no. 1, 1 January 2015 (2015-01-01), US, pages 90 - 99, XP055514324, ISSN: 1525-1578, DOI: 10.1016/ j.jmoldx.2014.08.005**
- **LISTVANOVA S ET AL: "Optimal kinetics for quantification of antigen-induced cytokines in human peripheral blood mononuclear cells by real-time PCR and by ELISA", JOURNAL OF IMMUNOLOGICAL METHODS, ELSEVIER SCIENCE PUBLISHERS B.V.,AMSTERDAM, NL, vol. 281, no. 1-2, 1 October 2003 (2003-10-01), pages 27 - 35, XP004468177, ISSN: 0022-1759, DOI: 10.1016/S0022-1759(03)00267-9**

**(Cont. next page)**

Remarks:

The complete document including Reference Table(s) and the Sequence Listing(s) can be downloaded from the EPO website

**Description**

The present invention refers to methods of detecting an infection with pathogens causing tuberculosis comprising the steps of a) contacting a first aliquot of a sample of an individual with at least one antigen of a pathogen causing tuberculosis, b) incubating the first aliquot with the at least one antigen for about 15 to about 23 hours, c) adding a cell lysing reagent which is preferably able to stabilize RNA in a temperature range of about 0°C to about 40°C for about 2 h to about 48 h, d) performing an RNA extraction from the mixture obtained in step c), wherein RNA is extracted from the mixture obtained in step c) without any prior washing or separation steps, e) detecting in the first aliquot and in a second aliquot of the sample of the individual the markers IFNG and CXCL10 and at least one housekeeping gene using a 1-step or 2-step reverse transcription quantitative real-time polymerase chain reaction (RT-qPCR), wherein the second aliquot has not been incubated with the at least one antigen, and f) comparing at least one of the detected markers in the first aliquot with the detected markers in the second aliquot, preferably in a fold change analysis, more preferably in a classifier method. In addition, the present invention refers to a first and second kit for performing the methods according to the present invention or one or more single steps thereof.

Tuberculosis is a widespread infectious disease, which is caused by different strains of mycobacteria (in particular *Mycobacterium tuberculosis,* Mtb). It affects primarily the lung (pulmonary TB) with manifestations in other areas of the body such as lymph nodes, urinary tract, bones, joints and the gastrointestinal tract (extrapulmonary TB). According to estimates of the world health organisation (WHO) in 2014 approximately 1.7 million people died from tuberculosis. Thus, tuberculosis remains one of the three major deadly infectious diseases worldwide. In addition, worldwide approximately two billion humans are latently infected with the pathogen and the number increases by approximately 10.4 million new cases per year (WHO Global Tuberculosis Report 2017).

During lifetime, approximately, 10-15 % of the latently infected immunocompetent individuals develop a treatment requiring active tuberculosis. Substantially higher numbers of reactivations are observed in patients with impaired immune function such as HIV patients.

Considering the lack of an effective, broadly protective vaccine, a rapid and reliable diagnosis of mycobacterial infection remains an important step to identify infected individuals and thus to perform differential diagnosis of the status of disease and to initiate appropriate, personalized treatment.

The currently available methods for the diagnosis of mycobacterial infections can be classified in three groups:

- patient anamnesis and clinical symptoms

- methods for direct pathogen detection

- methods for the detection of mycobacteria-specific cellular immune reactions

Besides patient anamnesis, X-ray examination and bacterial diagnostics remain centrial clinical methods for a comprehensive diagnosis of the status of tuberculosis.

X-ray examination: Till today, X-ray examination plays an important role in the detection of active tuberculosis and monitoring of therapy success. Beyond that this method provides important directions regarding the early diagnosis as well as the exclusion of treatment requiring tuberculosis at tuberculin skin test (TST) and/or interferon-gamma release (IGRA)-test positive contact persons. Advantages of these methods are the high sensitivity, however with reduced specificity.

Microscopy: Sputum microscopy allows a rapid evaluation of the infectivity of a patient on suspicion for pulmonary tuberculosis. Limitations of the method are the low sensitivity of 50 to 70%. In addition, the assay allows no discrimination between living and dead bacteria and no species allocation.

**Culture:** Direct detection of the pathogen by culture represents the gold standard for the diagnosis of an active tuberculosis with high sensitivity and specificity. However, the method suffers from the long time to result (available at least after 2 to 4 weeks).

**Nucleic amplification tests (NAT):** NAT such as the GeneXpert MTB/RIF test (Cepheid Inc., Sunnyvale, USA) are primarily used for indication examinations to confirm reasonable suspicion for tuberculosis in sputum-negative patients. In addition, NAT enables a rapid discrimination of mycobacteria from non-tuberculous mycobacteria in patients with microscopy-positive sputum. However, these tests show limitations in patients with low bacterial load and patients suffering from extrapulmonary tuberculosis; latter represent at least 15 to 20% of all tuberculosis cases. In addition, the test is not suitable for children, as for children the extraction of sputum (by coughing from the depth of the lung) is very difficult and painful. In addition, NAT are not suitable for the control of therapy success as these tests also detect DNA or RNA of non-viable bacteria.

**Immunological methods:** Besides methods for the direct detection of pathogens particularly in industrialized countries immunological detection methods gain increasing importance. These tests are based on the detection of Mtb polypeptide-specific immune reactions as indirect "host-response" marker for an infection with a mycobacterial pathogen. The most prominent representative is the tuberculin scin test (TST), which has already been applied as a diagnostic test for more than one century. This method is characterized by a high sensitivity but a limited specificity. For example cross reactivity with nontuberculous mycobacteria or a vaccination with nontuberculous mycobacteria or vaccination with the BCG (Bacille Calmette-Guerin)-vaccine strain can lead to false positive test results. Otherwise, TST results can be false negative in immunocompromized patients such as HIV patients or transplant patients treated with immunosuppressive substances. In addition, false negative test results can arise during the pre-allergic phase of infection and at severe courses of a general disease. Thus, a negative TST result does not exclude the presence of tuberculosis.

In contrast to TST the since 2005 commercially available interferon-gamma release tests (IGRAs) allow for the first time a differentiation of infected patients from vaccinated individuals. The test bases on the specific detection of *M. tuberculosis* polypeptide-reactive memory T cells, which are generated within the course of a mycobacterial infection. Renewed contact with *M. tuberculosis* polypeptides results in a specific reactivation of these cells coinciding with the production of characteristical marker cytokines.

The IGRA tests are based on the stimulation of isolated blood cells or anticoagulated whole blood of a patient with preselected Mtb polypeptides and the subsequent determination of the number of marker cytokine (mostly IFN-g)-producing cells (T-Spot-TB test, (Oxford Immunotec Ltd., Oxford UK)) or the quantification of produced marker cytokine (e.g. IFN-g) by ELISA (Quantiferon-TB Gold in Tube (QFT-GIT), Qiagen, Hilden, Germany). Herein, the numbers of cytokine secreting cells or the concentrations of specifically secreted marker cytokines serve as an indirect immunological marker for the detection of mycobacterial infection.

Compared to the TST test the IGRA assays show subsequently described advantages: no significant distorsion of the test result by BCG vaccination or infection with almost all non-tuberculous mycobacteria (NTM). In addition, in contrast to the TST test performance of the *in vitro* IGRA assay does not stimulate of patient's immune system and thus to a falsification of subsequent measurements; in addition there is no need for a second visit to perform the assay.

One important limitation of both types of IGRA assays is the not satisfactory sensitivity and specificity, whereby widely disparate test results have been reported in different studies. A meta-analysis based on the evaluation of 157 studies published in 2017 by Doan and coworkers reported test sensitivities for the TST, QFT-GIT and the T-Spot-TB test in immunocompetent adults for the detection of latent tuberculosis sensitivities of 84, 52 and 68% and specificities of 97, 97 and 93%, respectively. In addition, in children the TST shows higher test sensitivity when compared to the QFT-GIT. In immunologically compromised individuals the TST and QFT-GIT show only a weak sensitivity with a high sensitivity (Doan et al. (2018) PLOS ONE 12(11):e0188631).

In the field of infection recognition (discrimination of active disease and latent infection on the one hand versus patients without contact with mycobacterial pathogens on the other hand) a meta-analysis reports IGRAs to have sensitivities / specificities in a range of 73-83% and 49-79%, respectively (Sester et al. (2011) Eur. Resp. J. 37:100; World Health Organization, Tuberculosis IGRA TB Test Policy Statement, 2011).

Thus, there exists a need for a method, which allows a more reliable and automatable detection of mycobacterial infections.

In addition, within the last decade novel molecular immunodiagnostic tests have been developed based on RT-qPCR-based quantification of markers, which are produced by tuberculosis-specific memory T cells and/or antigen presenting cells in response to stimulation with tuberculosis antigens (WO2008028489A3, WO2012037937A2). Herein, relative quantification of CXCL10 mRNA by qPCR as claimed in WO2008028489A3 is almost equally efficient in detection of mycobacterial infection as the commercial (QFT-GIT) test (Blauenfeld et al. (2014) PLOS ONE 9:e105628). Divergent from the method described in the patent application WO2012037937A2 the present invention describes a RT-qPCR-based method for the discrimination of active tuberculosis and latent mycobacterial infection from non-infected individuals.

Kim et al. (2015) J. Mol. Diagn. 17(1):90-99 discloses a method of diagnosing TBC by stimulating whole blood with a commercially available mycobacterial antigen mix and measuring the mRNA of cytokines.

The problem to be solved by the present invention was thus to provide a more specific and/or sensitive method for detecting infection with pathogens causing tuberculosis. A further problem to be solved by the present invention was the provision of a method for detecting infection with pathogens causing tuberculosis which can be partly or fully automatized. A further problem to be solved by the present invention was the provision of a method in which a blood sample can be directly used for detection. A further problem to be solved by the present invention was the provision of a method allowing a more time- and cost-effective test result, in particular adapted to the usual process of obtaining a sample of an individual at a doctor's office and shipping the sample to a laboratory for further analysis. A further problem to be solved was the provision of a more stable antigen for stimulation. A still further problem to be solved by the present invention was the omission of a washing/separation step after the stimulation of the sample and prior to RNA extraction for reducing the required workload for performing the test. A still further problem to be solved by the present invention was the reduction of the required blood sample volume for performing the test.

The problem underlying the present invention is solved by the subject matter defined in the claims.

The following figures serve the purpose of illustrating the invention.

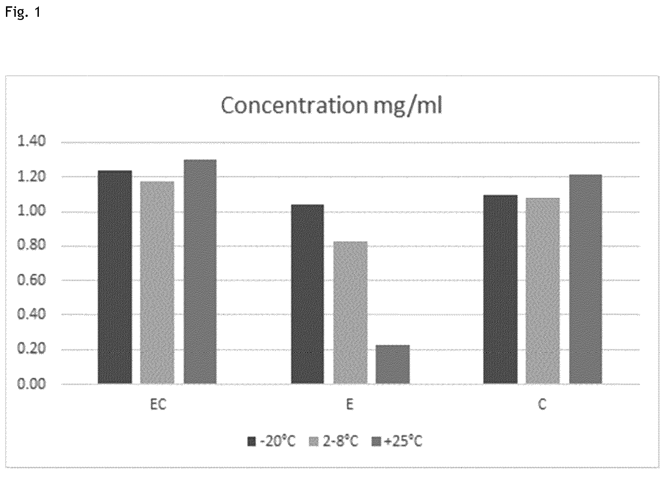

Figure 1 shows a column diagram representing the concentration of the antigens ESAT6 / CFP-10 heterodimer (EC), ESAT6 (E) and CFP-10 (C) at month 18 post storage at -20°C, 4 to 8°C and 25°C. The concentration of the antigens is determined by UV-Spectrophotometry at 280 nm. The starting concentration of each antigen was 1 mg/ml.

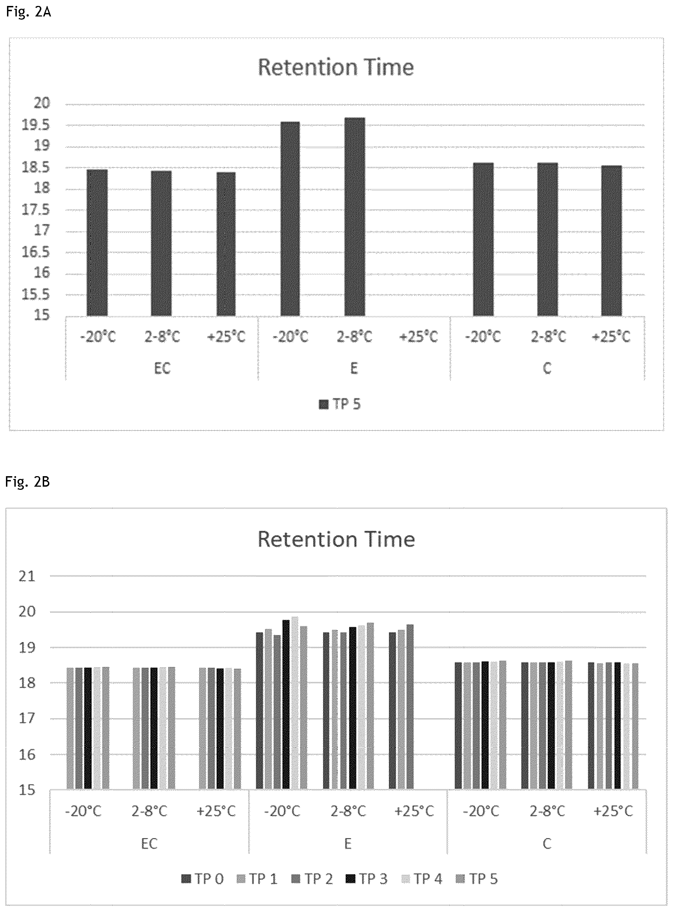

Figure 2 shows column diagrams representing the results of a size exclusion high performance liquid chromatography (SE-HPLC) analysis of the antigens ESAT6 (E) and CFP-10 (C) monomers and ESAT6/CFP-10 heterodimer (EC). In Figure 2A the column diagram represents the results of the SE-HPLC analysis of the antigens 18 months after storage at -20°C, 2 to 8°C and 25°C. Figure 2 B shows the column diagram representing the results of the SE-HPLC analysis of the antigens at different time-points of storage, i.e. TP 0 = 0 months, TP 1 = 1 months, TP 2 = 3 months, TP 3 = 6 months, TP 4 = 9 months, TP 5 = 18 months of storage at the indicated temperatures -20°C, 2 to 8°C and 25°C.

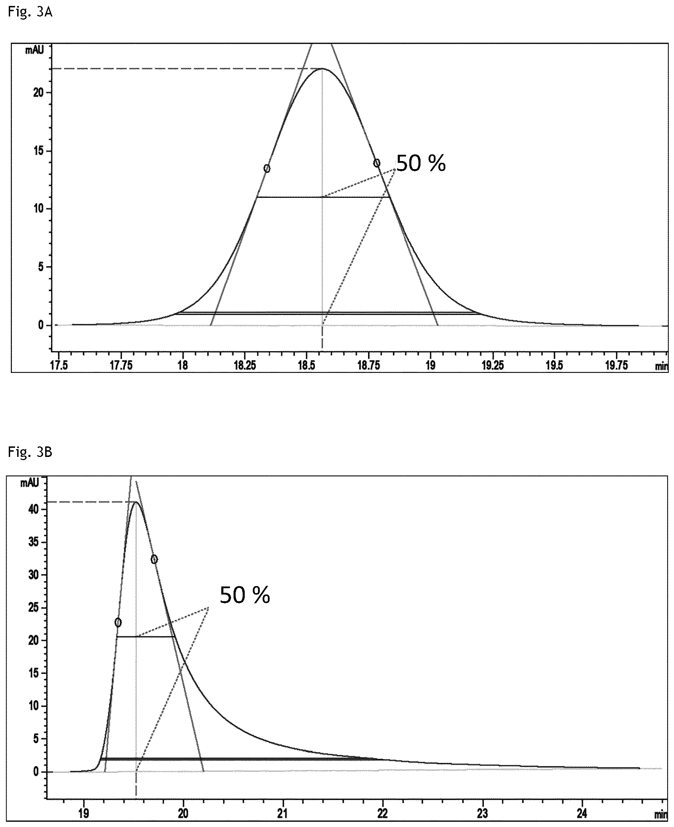

Figure 3 shows graphical representations of examples for peak shapes and the calculation of peak width and symmetry. Peak width is calculated at 50% height, peak symmetry is calculated based on the area before and after of the middle of the peak. Figure 3A shows a symmetric peak with symmetry = 1. Figure 3 B shown a peak with tailing with symmetry below 1.

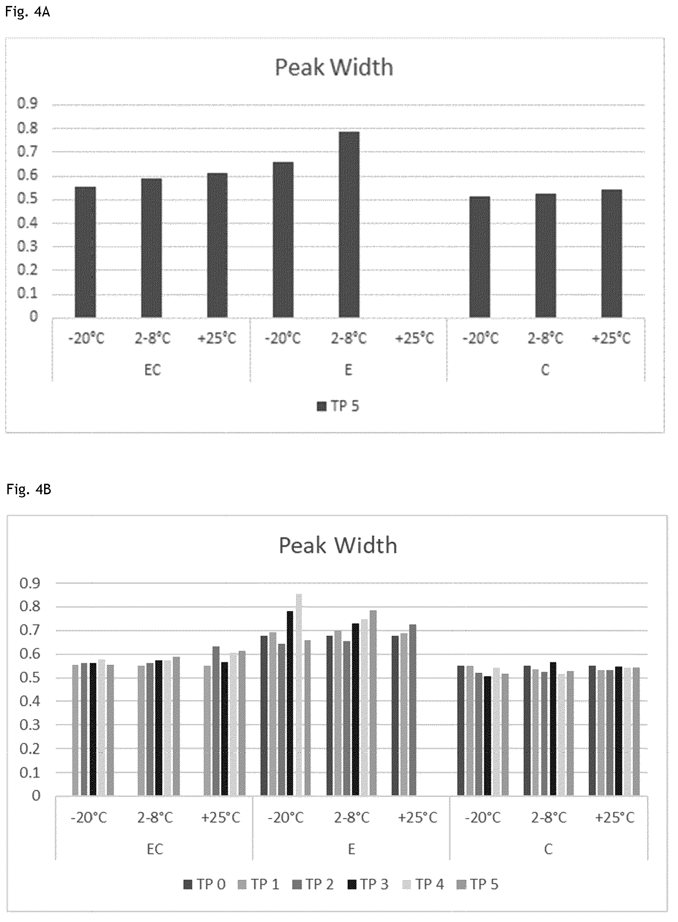

Figure 4 shows column diagrams representing the results of peak analysis by analytical size exclusion chromatography (SEC) of the antigens ESAT6 (E), CFP-10 (C) and the heterodimer of ESAT6 and CFP-10 (EC). In figure 4 A the column graph represents the peak width of the antigens 18 months after storage at -20°C, 2 to 8°C and 25°C. In figure 4B the column diagram represents the peak width of the antigens at different time-points of storage, i.e. TP 0 = 0 months, TP 1 = 1 month, TP 2 = 3 months, TP 3 = 6 months, TP 4 = 9 months, TP 5 = 18 months of storage at the indicated temperatures -20°C, 2 to 8°C and 25°C.

In the context of the present invention an "antigen" is preferably understood to be a protein, a multimer, as e.g. a heterodimer, in particular a multimer or heterodimer of two proteins, a polypeptide or a peptide, wherein said protein, multimer, polypeptide or peptide preferably encodes at least a part of or a complete pathogen causing tuberculosis. In addition, an antigen may be understood to be a RNA, DNA or an expression plasmid, wherein said nucleic acids encode at least a part, preferably a peptide, polypeptide or protein of least a part of or a complete pathogen causing tuberculosis. Preferably, the antigen is an antigen of a wild type pathogen causing tuberculosis but not of attenuated M. tuberculosis strains used for vaccination, in particular not of the BCG (Bacille Calmette-Guerin)-vaccine strain.

The term "sensitivity" as used herein refers preferably to the % of patients with active tuberculosis and latent mycobacterial infection (defined as "infected") that are correctly classified as infected.

The term "specificity" as used herein refers preferably to the % of individuals with no previous contact with a pathogen causing tuberculosis as e.g. mycobacteria (defined as "non-infected") that are correctly classified as non-infected.

In the context of the present invention the term "polypeptide" is preferably understood to be a polymer of amino acids of any length. The phrase "polypeptide" comprises also the terms target epitope, epitope, peptide, oligopeptide, protein, polyprotein and aggregate of polypeptides. Furthermore, the expression "polypeptide" also encompasses polypeptides, which exhibit posttranslational modifications such as glycosylations, acetylations, phosphorylations, carbamoylations and similar modifications. In addition, the expression "polypeptide" is understood to refer also to polypeptides, which exhibit one or more analogues of amino acids, such as for example non-natural amino acids, polypeptides with substituted linkages as well as other modifications known in the prior art, irrespective thereof, whether they occur naturally or are of non-natural origin.

The term "heterodimer" as used herein refers preferably to a heterodimer provided as a heterodimer and not to a heterodimer which is spontaneously formed in a mixture of the single monomers of the heterodimer.

In the context of the present invention "reverse transcription quantitative real-time polymerase chain reaction, RT-qPCR" is preferably understood to be a method, which is based on the conventional polymerase chain reaction (PCR). In addition, RT-qPCR allows, besides amplification, in addition also a quantification of the target mRNA. For this purpose the total RNA is isolated from the material to be examined and incubated with an antigen and is isolated in comparison from unstimulated material or material incubated with an irrelevant antigen, and is then transcribed into cDNA in a subsequent reverse transcription reaction. By using specific primers the target sequence is then amplified in the qPCR. For quantification of the target sequence several methods may be applied.

The most simple way of quantification in RT-qPCR is using intercalating fluorescent dyes, such as SYBR green or EVA green. These dyes fit themselves in the double stranded DNA molecules, which arise during the elongation of the specific products. The detection always takes place at the end of the elongation by detecting the emitted light after excitation of the fluorescent dye. With increasing amount of PCR product more dye is incorporated, thus the fluorescent signal increases.

A further possibility of quantification in RT-qPCR is the use of sequence specific probes. There are hydrolysis (TaqMan) or hybridisation (Light-Cycler) probes. Hydrolysis probes are labelled at the 5' end with a fluorescent dye and at the 3' end with a so-called quencher. Due to the spatial proximity to the reporter dye the quencher is responsible for the quenching of the fluorescence signal and is cleaved off during the synthesis of the complementary DNA in the elongation phase. As soon as the fluorescent dye is excited with a light source at the end of the elongation, light of a specific wave length is emitted, which may be detected.

Hybridisation probe systems consist of two probes, which bind to a target sequence next to each other. Both probes are labelled with a fluorescent dye. With a light source the first fluorescent dye at the 5' end of the first probe is excited. The emitted light is then transferred via fluorescence resonance energy transfer (FRET) to the second fluorescent dye at the 3' end of the second probe. Thereby the dye is excited, whereby light of a specific wave length is emitted, which may be detected. If in the course of the elongation of the complementary strand of the target sequence the first probe is degraded by the polymerase, the FRET may no more take place and the fluorescence signal subsequently decreases. In contrast to the afore-mentioned methods the quantification thus occurs here always at the beginning of the elongation process.

Frequently used fluorescent dyes are for example medical fluorophore 1 (MF1), medical fluorophore 2 (MF2), 7-Amino-4-methylcoumarin (Merck), Fluorescein (Merck), Cyanine 3 (Cy3) (ThermoFisher), Cyanine 5 (Cy5) (Thermo-Fisher), europium (TCI), bodipy, dansyl, naphtalene, ruthenium, tetramethylrhodamine, 6-carboxyfluorescein (6-FAM), VIC (BioCat), YAK, rhodamine, TET, 6-carboxy-4',5'-dichloro-2',7'-dimethoxyfluorescein (6-JOE), HEX (ThermoFisher Cat. No. 403170), NED (ThermoFisher), ROX (Biomol GmbH, Cat. No. 400), 5-carboxytetramethoylrhodamine (5-TAMRA) (Merck, CAS No. 91809-66-4), and Texas Red (ThermoFisher). Frequently used quenchers are for example 5-carboxytetramethoylrhodamine (5-TAMRA) (Merck, CAS No. 91809-66-4), methyl red, dabcyl, eclipse, Tide Quencher 2 (TQ2) (Biomol GmbH, Cat. No. 2200), BHQ1 (Jena Bioscience), Tide Quencher 3 (TQ3) (Biomol GmbH, Cat. No. 2228), BHQ2 (Jena Bioscience) or BlackBerry® Quencher 650 (BBQ650).

The term "real-time" refers preferably to a distinct measurement within each cycle of PCR, i.e. in "real-time". The increase of the so-called target sequence correlates herein with the increase of the fluorescence from cycle to cycle. At the end of a run, which usually consists of several cycles, the quantification is then carried out in the exponential phase of the PCR on a basis of the obtained fluorescents signals. Hereby, the measurement of the amplification is usually done via $C_q$ (quantification cycle) values, which described the cycle, in which the fluorescence rises for the first time significantly above the background fluorescence. The $C_q$ value is determined on the one hand for the target nucleic acid and on the other hand for the reference nucleic acid. In this way it is possible to determine absolute or relative copy numbers of the target sequence.

In a preferred embodiment of the invention the normalisation of the gathered real-time PCR data (real-time PCR data) is performed by using a fixed reference value, which is not influenced by the conditions of the experiment, in order to achieve a precise gene expression quantification. For this purpose, the expression of a reference gene is also measured in order to perform a relative comparison of amounts.

In the context of the present invention the expression reference gene may be understood as a sequence on mRNA level as well as on the level of genomic DNA. These may also be non-transcriptional active under the stimulation conditions according to the present invention or they correspond to non-coding DNA regions of the genome. According to the invention a reference gene may also be a DNA or RNA added to the target gene sample. The highest criterion of a reference gene is that it is not altered in the course of the stimulation and by the conditions of the inventive method. The experimental results may thus be normalized with respect to the amount of template used in different samples. The reference gene allows thus the determination of the relative expression of a target gene. Examples for reference genes, which may also be called housekeeping genes, are 60S acidic ribosomal protein P0 (RPLPO), β-actin, glyceraldhyde-3-phosphate-dehydrogenase (GAPDH), porphobilinogen deaminase (PBGD), hypoxanthin-phosphoribosyl-transferase 1 (HPRT1); TATA box binding protein (TBP)-associated factor, RNA polymerase I, A (TAF1A) or tubulin.

The term "% sequence identity" is generally understood in the art. Two sequences to be compared are aligned to give a maximum correlation between the sequences. This may include inserting "gaps" in either one or both sequences, to enhance the degree of alignment. A % identity may then be determined over the whole length of each of the sequences being compared (so-called global alignment), that is particularly suitable for sequences of the same or similar length, or over shorter, defined lengths (so-called local alignment), that is more suitable for sequences of unequal length. In the above context, an amino acid sequence having a "sequence identity" of at least, for example, 95% to a query amino acid sequence, is intended to mean that the sequence of the subject amino acid sequence is identical to the query sequence except that the subject amino acid sequence may include up to five amino acid alterations per each 100 amino acids of the query amino acid sequence. In other words, to obtain an amino acid sequence having a sequence of at least 95% identity to a query amino acid sequence, up to 5% (5 of 100) of the amino acid residues in the subject sequence may be inserted or substituted with another amino acid or deleted. Methods for comparing the identity and homology of two or more sequences are well known in the art and may for example be performed by a BLAST analysis. In addition, if reference is made herein to a sequence sharing "at least" at certain percentage of sequence identity, then 100% sequence identity are preferably not encompassed.

The term "equivalents or variants" in respect of a buffer specified by its manufacturer and a catalog number as used herein refers to a buffer comprising basically the same components as the specified buffer. The weight proportion of the single components may differ by about 30%, 20%, 10% or about 5%. Basically the same means in particular that the components responsible for the desired effect of the buffer are the same. In particular, an equivalent or variant buffer shows the same desired chemical or physical effects as the specified buffer. Said effects may preferably range about 80% to about 120 %, or about 90% to about 110%, or about 95% to about 105% of the effects of the specified buffer.

In a first object of the present invention it is envisaged to provide an *in vitro* method of detecting an infection with pathogens causing tuberculosis, the method comprises the steps of:

a) contacting a first aliquot of a sample of an individual with at least one antigen of a pathogen causing tuberculosis, in particular wherein a heterodimer of two antigens, more preferably a heterodimer of ESAT-6 and CFP-10, is added to the first aliquot or wherein the first aliquot is comprised in a container comprising a heterodimer,
b) incubating the first aliquot with the at least one antigen for about 15 to about 23 hours,
c) adding a cell lysing reagent which is able to stabilize RNA in a temperature range of about 0°C to about 40°C, more preferably of about 2°C to about 30°C, or of about 15°C to about 30°C, or of about 2°C to about 8°C, or of about 15°C to about 25°C, or about of 18°C to about 30°C for about 2 h to about 48 h, more preferably for about 12 h to about 48 h more preferably for about 2 h to about 72 h, more preferably for about 12 h to about 72 h,
d) performing an RNA extraction from the mixture obtained in step c), preferably an automated RNA extraction, wherein RNA is preferably extracted from the mixture obtained in step c) without any prior washing or separation steps,
e) detecting in the first aliquot and in a second aliquot of the sample of the individual the markers IFNG and CXCL10 and at least one housekeeping gene using a 1-step or 2-step reverse transcription quantitative real-time polymerase chain reaction (RT-qPCR), wherein the second aliquot has not been incubated with the at least one antigen, and
f) comparing all of the detected markers in the first aliquot with the detected markers in the second aliquot, preferably in a fold change analysis, more preferably in a classifier method,

wherein the sample of the individual in step a) is whole blood, in particular anti-coagulated whole blood, or a purified or isolated PBMC population, lymph or a bronchial lavage and wherein the cell lysing reagent which is able to stabilize RNA comprises a detergens such as such as Triton X 100, Tween 20, Tween 80, 3 [(3 Cholamidopropyl)dimethylammonio] 1 propanesulfonate (CHAPS), urea and/or n-Dodecyl-β-D-Maltopyranoside (DDM), a chaotropic agent such as a guanidinium lysis buffers comprising preferably guanidinium isothiocyanate and/or guanidinium-HCl and an antioxidant such as DTT, Dithioerythritol (DTE), Tris(2-carboxyethyl)phosphin (TCEP) and/or β-Mercaptoethanolopic agent.

The *in vitro* method of detecting an infection with pathogens causing tuberculosis according to the present invention is preferably an *in vitro* method for differentiating individuals being infected with pathogens causing tuberculosis and individuals being uninfected with pathogens causing tuberculosis. The method according to the present invention provides an improved detection of infection with tuberculosis pathogens, especially of individuals with active tuberculosis. The test allows the diagnosis of infection with tuberculosis pathogens and their differentiation from individuals without contact with tuberculosis pathogens. Individuals without contact with tuberculosis pathogens preferably include non vaccinated individuals without contact with tuberculosis pathogens and individuals being vaccinated against tuberculose, as e.g. BCG vaccinated individuals, which had no further contact with tuberculosis pathogens. Both people with latent infection and patients with active disease are detected. In a preferred embodiment also actively infected individuals under initiation of antibacterial therapy, e.g. with Rifampicin, are detected as having been in contact with a pathogen causing tuberculosis. The method according to the present invention does not allow distinguishing between individuals having a latent infection and individuals having active tuberculosis.

The method according to the present invention allows an improved detection of individuals with latent infection with pathogens causing tuberculosis and patients suffering from active tuberculosis and the discrimination from non vaccinated and preferably vaccinated, preferably BCG-vaccinated individuals, with no contact with a pathogen causing tuberculosis. This methodology has improved performance parameters compared to the commercially available tuberculin skin (PPT) and interferon gamma release (IGRA) tests and provides some operational advantages such as high analytical accuracy, rapid availability of test result and suitability for fully automated workflows. In addition, the present invention provides examples for such automated workflows which are amongst others comfortably adapted to the usual workflow of obtaining a sample from an individual, shipping the sample to a labatory and analyzing the sample in the labatory as fast and efficient as possible. In particular, the method according to the present invention provides that the night after obtaining the sample from an individual may directly be used for performing the stimulation step (step b)). Moreover, the method according to the present invention provides that step a), b) and/or c) can e.g. be performed without needing special laboratory equipment or skills. It is forseen that steps a), b) and/or c) can e.g. be performed directly at the doctor's office. In step c) the sample is stabilized so that it can be stored and shipped up to 3 days at about room temperature to a laboratory for performing the remaining steps of the method according to the present invention. In addition, with the automated workflows provided herein the processing time (without step b)) can be reduced to about 4 to about 6 hours, wherein the hands-on time (i.e. the time a laboratorian needs to use its hands for performing the method steps of the present invention) can be reduced to about 1.5 hours to about 4 hours.

Unexpected findings were that the antigens ESAT-6 and CFP-10 can be provided and used as a heterodimer much more stable and efficient than if they are provided and stored separately. Surprisingly, the use of a heterodimer of ESAT 6 and CFP-10 instead of seperatly added single antigens does not reduce the specifcy and sensivity of the method of detection but seems to improve it.

Further unexpected findings were that in step c) a cell lysing reagent can be used which is additional able to stabilize RNA, in particular at a temperature of about 15°C to about 30°C for about 1 to 2 days, more preferably for about 1 to 3 days. This allows the shipment of stabilized RNA for further analysis to another laboratory preferably without any need for cooling. Unexpectively, said lysing reagent can be added directly to the stimulated blood sample obtained in step b) of the method according to the present invention without any need of performing any washing or separation steps. Even more surprisingly, RNA can be directly extracted from the mixture obtained in step c), and thus directly from the stimulated whole blood sample to which the cell lysing reagent have been added, without any prior washing or separation steps.

In a preferred embodiment the detection of an infection with pathogens causing tuberculosis is a differentiation of individuals having been in contact with a pathogen causing tuberculosis and individals having not been in contact with a pathogen causing tuberculosis. Individuals having been in contact with pathogens causing tuberculosis comprise preferably individuals having acute tuberculosis, active tuberculosis, which preferably requires treatment, latent infection with pathogens causing tuberculosis and individuals in which tuberculosis have been successfully treated i.e. the pathogens causing tuberculosis have been successfully killed or combated by therapy. In a preferred embodiment also actively infected individuals under initiation of antibacterial therapy e.g. with Rifampicin are detected as having been in contact with a pathogen causing tuberculosis. Preferably, individals having not been in contact with pathogens causing tuberculosis comprise individuals having been vaccinated against tuberculosis, in particular individuals having been vaccinated with the Bacillus Calmette-Guérin (BCG) vaccination strain. Such individuals may also be called BCG-vaccinated individuals. The individual may be a human or an animal.

According to the invention it is contemplated that the method of detecting an infection with pathogens causing tuberculosis comprises the step of providing a sample of an individual. Said sample is preferably a liquid sample as e.g. a whole blood sample. In the context of the present invention "providing" is understood to imply that an aliquot of the sample is already present in a container. "Providing" may also mean according to the invention, that the aliquot of the sample is directly provided from a patient, for instance by sampling blood.

The inventive method envisages that the first aliquot is stimulated with at least one antigen, while the second aliquot remains unstimulated. However, said aliquot may be incubated with a buffer, preferably PBS. However, said second aliquot may be incubated or even stimulated by a mock control. A mock treatment is a sham treatment of reaction or incubation approaches which serves as a control experiment. In a mock treatment the mock control is preferably treated in the same way as the parallel approach but without one or more active components. Said mock control may comprise antigens but no antigens of pathogens causing tuberculosis and/or no antigens causing the specific reaction which is caused by pathogens causing tuberculosis. All in all, it is thus envisaged, that the first and second aliquot are identical except for the contact with the antigen/s, i.e. the antigens of pathogens causing tuberculosis which are used in step (a) of the methods according to the present invention. However, instead of the antigen(s) of pathogens causing tuberculosis one ore more different antigens, which are not of pathogens causing tuberculosis and/or do not cause the specific reaction which is caused by pathogens causing tuberculosis may be added to the second aliquot e.g. for stimulating the components of the second aliquot. Preferably, the first and second aliquot are identical except for the added stimulants and antigens, respectively. Hence, the second unstimulated aliquot serves as a kind of calibrator. The quantification is thus performed relative to the calibrator. For the determination and quantification of the marker it is envisaged, that the amount of marker in the first stimulated aliquot is divided by the amount of the marker in the second unstimulated aliquot. Thus, an n-fold difference in amount of the marker of the first stimulated aliquot relative to the calibrator, i.e. the second unstimulated aliquot, is detected. The inventive method represents a method which is exclusively carried out *ex vivo*.

Preferably, the pathogen causing tuberculosis is preferably *Mycobacterium tuberculosis, Mycobacterium bovis* (*ssp. bovis* und *caprae*), *Mycobacterium africanum, Mycobacterium microti, Mycobacterium canetti* and *Mycobacterium pinnipedii.*

In a preferred embodiment the sample is blood, lymph, or a bronchial lavage. The blood is prefably whole blood or anticoagulated whole blood. Also preferred are embodiments in which the sample is a purified PBMC population, preferably a PBMC population isolated from whole blood, or cells isolated from a bronchial lavage. Cells isolated from a bronchial lavage may for example be obtained by applying density gradient centrifugation *using* Ficoll-Paque media. Isolated cells may be resuspended and optionally cultured in a suitable medium as e.g. serum-free medium or serum containing medium. For providing a test result most cost and time efficient the sample of the individual is preferably a whole blood sample, in particular an anticoagulated whole blood sample.

The sample of an individual can be a previously obtained from a human or an animal patient. Preferably, the method according to the present invention is performed about up to 8h, 12h, 16h, 20h, 24h, 40h or 48h after the sample of the individual was obtained and prior to step a), respectively. In particular, it is performed about 0 to about 48 hours, more preferably about 0 to about 40 hours, or about 0 to about 24 hours or about 0 to about 20 hours, or about 0 to about 16 hours, or about 0 to about 12 hours, or about 0 to about 8 hours after the sample of the individual was obtained. It may also be performed about 2 to about 8 hours, about 2 to about 4 hours, about 2 to about 6 hours, about 2 to about 48 hours, about 2 to about 40 hours, about 2 to about 24 hours, about 2 to about 20 hours, about 2 to about 16 hours, about 2 to about 12 hours, or about 2 to about 8 hours after the sample of the individual was obtained. After the sample was obtained from the individual, the sample is preferably stored at a temperature above 0°C, more preferably at a temperature of about 0°C to about 50°C, about 4°C to about 40°C, about 10°C to about 35°C or about 16°C to about 30°C, or about 18°C to about 25°C, about 2°C to about 10°C or at about room temperature.

The method according to the present invention can be performed by using a small blood sample volume which is very convenient for the individual from which it is obtained. The volume of the sample of the individual in the first, second and optionally third aliquot is preferably about 0.2 mL to about 3 mL or about 0.2 mL to about 2.5 mL, or about 0.2 mL to about 2 mL, or about 0.5 mL to about 1.5 mL, or about 0.6 mL to about 1.2 mL, or about 0.7 mL to about 1.1 mL, or about 0.8 mL to about 1 mL.

In a preferred embodiment the at least one antigen of a pathogen causing tuberculosis is a peptide, oligopeptide, a polypeptide, a protein, an RNA or a DNA. According to the invention the antigen may furthermore preferably be a fragment, a cleavage product or a piece of an oligopeptide, of a polypeptide, of a protein, of an RNA or of a DNA. In a further preferred embodiment, the at least one antigen of a pathothen causing tuberculosis is a protein, in particular having a molecular weight of about 4 kDa to about 100 kDa, or about 5 kDa to about 90 kDa.

In a preferred embodiment of the method according to the present invention step (a) comprises contacting a first aliquot of a sample of an individual with two, three, four, five, six, seven, eight, nine or ten antigens of a pathogen causing tuberculosis. The aliquot in step (a) may also be contacted with 11, 12, 13, 14, 15, 16, 17, 18, 19, 20, 21, 22, 23, 24, 25, 26, 27, 28, 29 or 30 or with a pool of antigens comprising about 10 to about 100, about 20 to about 100, about 30 to about 100, about 40 to about 100 or about 50 to about 100 antigens. If more than one antigen is used, all antigens are preferably distinct antigens. The distinct antigens may be derived from one or more different pathogens causing tuberculosis. They may also derive from the same pathogen causing tuberculosis. If 3 or more than 3 distinct antigens are used some of the antigens may derive from the same pathogen and the other may derive from different pathogens causing tuberculosis. A pool of antigens comprises preferably peptides as antigens.

In a preferred embodiment the at least one antigen and optionally the further antigens as described above are selected from the group consisting RD-1 antigens, ESAT-6, CFP-10, TB7.7, Ag 85, HSP-65, Ag85A, Ag85B, MPT51, MPT64, TB10.4, Mtb8.4, hspX, Mtb12, Mtb9.9, Mtb32A, PstS-1, PstS-2, PstS-3, MPT63, Mtb39, Mtb41, MPT83, 71-kDa, PPE68 and LppX. Especially preferred antigens are ESAT-6, CFP-10, TB 7.7, Ag 85, HSP 65 and other RD-1 antigens. RD1-1 antigens are preferably the following antigens: Rv3871, Rv3872, Rv3873, CFP-10, ESAT-6, Rv3876, Rv3878, Rv3879c and ORF-14.

Alternatively or in addition, the antigens may be also selected from the group consisting H1-hybrid, AlaDH, Ag85B, Pst1S, Ag85, ORF-14, Rv1989c, Rv0134, Rv0222, Rv0934, Rv1256c, Rv1514c, Rv1507c, Rv1508c, Rv1511, Rv1512, Rv1516c Rv1766 Rv1769 Rv1771, Rv1860, Rv1974 Rv1976c Rv1977, Rv1980c, Rv1982c, Rv1984c, Rv1985c, Rv2031c, Rv2074, Rv2780, Rv2873 Rv3019c, Rv3120, Rv3615c Rv3763, Rv3871, Rv3872, Rv3873, Rv3876, Rv3878, Rv3879c, Rv3804c, Rv3873, Rv3878, Rv3879c or a polypeptide mixture, such as tuberculin PPD.

Alternatively or in addition, the antigens may be selected from the group consisting of Rv3879c, Rv1508c, Rv3876, Rv1979c, Rv2655c, Rv1582c, Rv1586c, Rv3877, Rv2650c, R1576c, Rv1256c, Rv3618, Rv2659, cRv1770, Rv1771, Rv1769, Rv3428c, Rv1515c, Rv1511, Rv1512, Rv1977, Rv1985c, Rv0134, Rv1509, Rv3427c, Rv2646, Rv1041, cRv1507c, Rv1980c, Rv1514c, Rv1190, Rv3878, Rv1969, Rv1975, Rv1968, Rv1971, Rv3873, Rv2652c, Rv2651c, Rv1585c, Rv1577c, Rv1972, Rv1507A, Rv1506c, Rv1966, Rv1973, Rv1573, Rv1578c, Rv1974, Rv1575, Rv2645, Rv1987, Rv1970, Rv2074, Rv1976c, Rv2073c, Rv2810c, Rv1581c, Rv3136A, Rv2548A, Rv3098A, Rv2231A, Rv2647, Rv1772, Rv1508A, Rv2658c, Rv1767, Rv2063A, Rv1954, ARv1583c, Rv2656c, Rv0724A, Rv3875, Rv2348c, Rv0222, Rv2653c, Rv1580c, Rv1579c,Rv1766, Rv1366A, Rv3874, Rv0061c, Rv1768, Rv0397A, Rv1991A, Rv2274A, Rv3617, Rv1574, Rv3350c, Rv1984c, Rv2801A, Rv3872, Rv2657c, Rv1983, Rv2142A, Rv1967, Rv2862A, Rv3190A, Rv2237A, Rv2468A, Rv1982A, Rv1982c, Rv1584c, Rv0691A, Rv2395A, Rv2654c, Rv2231B, Rv1257c, Rv2395B, Rv1516c, Rv0186A, Rv0530A, Rv0456B, Rv3120, Rv3738c, Rv3121, Rv3426, Rv3621c, Rv0157A, Rv2349c, Rv1965, Rv3508, Rv3514, Rv0500B, Rv1978, Rv2350c, Rv2351c, Rv1986, Rv3599c, Rv2352c, Rv1255c, Rv2356c, Rv2944, and Rv3507.

Particularly preferred is an embodiment of the present invention, wherein step (a) comprises contacting a first aliquot of a sample of an individual with at least ESAT-6. In a further preferred embodiment, the sample of the individual is contacted with at least two antigens, in particular with ESAT-6 and CFP-10. Also, particularly preferred is an embodiment of the present invention, wherein step (a) comprises contacting a first aliquot of a sample of an individual with a heterodimer of two antigens, more preferably with a heterodimer of ESAT-6 and CFP-10. Contacting the first aliquot of a sample with a heterodimer of two proteins such as ESAT-6 and CFP-10 has the advantage that said heterodimer can be more easily isolated and stored than each protein alone. More particularly, ESAT6 becomes more soluble if it is mixed with CFP-10 to form a heterodimer. Thus, a heterodimer of ESAT-6 and CFP-10 can be provided with a higher yield and is better to handle. The heterodimer of ESAT-6 and CFP-10 may for example be obtained as described in Reichelt et al. (Protein Expression and Purification 46 (2006) 483-488). In particular the heterodimer may be obtained by expressing the proteins separately, performing a first purification step for each protein followed by a washing step. Subsequently, ESAT-6 and CFP-10 may be subjected to further separate purification and washing steps or they may firstly be mixed and the mixture is subjected to further purification and washing steps. If they are separately purified the purified proteins are mixed. In both mixtures ESAT-6 and CFP-10 are preferably used in equimolar concentrations. For purifying and washing the proteins a combination of affinity chromatography via e.g. immobilized metal affinity chromatography, ion exchange, and size exclusion chromatography may be used.

The protein ESAT-6 is not very stable on its own and difficult to purify and store. The inventors have surprisingly found that it is much more efficient to produce ESAT-6 combined with CFP-10 as said heterodimer. A respectively obtained heterodimer of ESAT-6 and CFP-10 is in fact easier to purify, more stable and can be stored for longer periods of time. Surprisingly, heterodimer works just as well or even better. In particular, the heterodimer of ESAT-6 and CFP-10 obtained as outlined above can be stored in a temperature range of about 2°C to about 8 °C for about a year or up to a year or in a temperature range of about 15°C to about 30°C or at room temperature for up to 1, 2, 3, 4, 5, 6 or 7 days.

If the first aliquot is comprised in a container comprising a heterodimer this preferably means that the first aliquot is present in a container previously provided with the heterodimer. A container previously provided with the heterodimer is for example a container (such as a blood sampling tube or a Heparin tubes) precoated with the heterodimer. Preferably, the inner surface of said container is precoated with the heterodimer. The heterodimer may for example be released from the inner surface by slight shaking. In a further preferred embodiment, step a) is performed in blood sampling tube (e.g. a Heparin tube) which is precoated with the at least one antigen, more preferably with a heterodimer of two antigens.

In a further embodiment, step a) is performed by applying a first aliquot of an anti-coagulated whole blood sample to a container (e.g. a tube) and adding the at least one antigen, preferably the heterodimer, to the first aliquot.

In a preferred embodiment of the present invention the period of time for contacting in step a) and incubation in step b) is about 15 to about 23 hours or over night. The period of time for contacting in step a) and incubation in step b) is the time during which the sample of the individual is contacted and thus stimulated with the at least one antigen. Preferably, the time period for the stimulation over night or in a time period of about 15 hours to about 23 hours is combined with a time period of less than or equal to 8 hours, 12 hours, 16 hours, 20 hours, 24 hours, 40 hours or 48 hours after the sample of the individual was obtained.

Steps a and/or b) are preferably performed at a temperature of about 30°C to about 40°C, more preferably of about 35°C to about 39°C, most preferably at about 37°C.

The cell lysing reagent added in step c) is preferably able to stabilize RNA in a temperature range of about 0°C to about 40°C, more preferably of about 2°C to about 30°C, or of about 15°C to about 30°C, or of about 2°C to about 8°C, or of about 15°C to about 25°C, or about of 18°C to about 30°C for about 2 h to about 72 h, more preferably for about 12 h to about 72h, more preferably for about 12 h to about 48 h. Preferably, it is also able to stabilize RNA in a temperature range of about -15°C to about -25°C for up to about 12 months, in a temperature range of about 2 to about 8°C for about 1 to about 3 days, in a temperature range of about - 20°C to about -70°C for up to 11 years and/or in a temperature range of about 2 to about 8°C for about 5 to about 8 days.

After the cell lysing reagent (which is preferably able to stabilize RNA) is added to the stimulated sample obtained in step b) the mixture of step c) is preferably stored prior to step d) in a temperature range of about 0°C to about 40°C, more preferably of about 2°C to about 30°C, or of about 10°C to about 40°C, or of about 15°C to about 30°C, or of about 2°C to about 8°C, or of about 15°C to about 25°C, or about of 18°C to about 30°C for about 2 h to about 72 h, more preferably for about 12 h to about 72h, more preferably for about 12 h to about 48 h. Alternatively, the mixture may be stored prior to step d) in a temperature range of about -15°C to about -25°C for up to about 12 months, in a temperature range of about 2 to about 8°C for about 1 to about 3 days, in a temperature range of about -20°C to about -70°C for up to 11 years or in a temperature range of about 2 to about 8°C for about 5 to about 8 days. The mixture may also be stored on dry ice or stored at about -80°C for further analysis. After the addition of the cell lysing reagent the mixtures is preferably shortly vortexted, e.g. for about 2 to about 10 seconds or for about 5 seconds.

The term "store" may thereby comprise any packaging or shipment of the mixture, leaving the sample to stand or keeping the sample.

Preferably the mixture obtained in step c) is mixed, preferably for about 5 to about 10 seconds at room temperature or at a temperature range of about 18°C to about 25°C.

The cell lysing reagent which is able to stabilize RNA comprises a detergens such as such as Triton X 100, Tween 20, Tween 80, 3-[(3-Cholamidopropyl)dimethylammonio]-1-propanesulfonate hydrate (CHAPS), urea and/or n-Dodecyl-$\beta$-D-Maltopyranoside (DDM), a chaotropic agent such as a guanidinium lysis buffers comprising preferably guanidinium isothiocyanate or guanidinium-HCl and an antioxidant such as DTT, Dithioerythritol (DTE), Tris(2-carboxyethyl)phosphin (TCEP), and/or β-Mercaptoethanolopic agent. The cell lysing reagent is preferably able to disrupt cells and to stabilize RNA, preferably simultaneously, preferably by inactivating RNases, in particular during cell lysis.

Whether a cell lysing reagent is able to stabilize RNA as required by the method according to the present invention can be easily verified by a person skilled in the art by adding a potenital cell lysing reagent to e.g. two whole blood samples, mixing the mixtures, storing the first mixtures at the minimum and the second mixture at the maximum temperature of the relevant temperature range to be tested (e.g. at 15°C and at 25°C if the temperature range of 15°C to 25°C is to be tested) and verifying e.g. the amount and the quality of RNA in the sample after the relevant time. The amount of RNA can e.g. be measured in spectrophotometric method using e.g. Nanodrop 1000. The quality of RNA can e.g. be verified in an electrophorese using e.g. the Experion system. As a reference for a suitable lysing reagent the RNA/DNA Stabilization Reagent for Blood/Bone Marrow (manufacturer: Roche; Cat. No. 11 934 317 001) may be used. If the cell lysing reagent to be tested results in the same or an equivalent amount and quality of RNA as the reference lysing reagent, then the tested cell lysing reagent is able to stabilize RNA as required by the method according to the present invention. An equivalent amount and quality of RNA thereby refers to an amount and quality of RNA of at least about 80%, 85%, 90% or 95% or in a range of about 80% to about 150%, about 85% to about 140%, about 90% to about 130%, or about 95% to about 120% of the amount and quality of RNA obtained by the reference lysing reagent.

In a preferred embodiment of the present invention the cell lysing reagent used in step c) of the method according to the present invention is able to stabilize RNA in the above listed temperature and time ranges from a 1 ml whole blood sample, in particular a 1 mL anti-coagulated whole blood sample, so that at least about 1.5, 1.6, 1.7 or 1.8 mg or about 1.5 to about 6 mg, more preferably about 1.6 to about 5.5 mg, more preferably about 1.7 to about 5 mg RNA can be extracted in step d).

A particulary preferred lysing reagent is the RNA/DNA Stabilization Reagent for Blood/Bone Marrow (manufacturer: Roche; Cat. No. 11 934 317 001) or an equivalent or variant thereof.

In a highly preferred embodiment of the present invention the cell lysing reagent is able to stabilize RNA, wherein the cell lysing reagent does not require any washing or separation (e.g. by centrifugation) or a combiantion of separation (e.g. by centrifugation) and washing steps steps prior to step d). This means that RNA is extracted from the mixture obtained in step c) without any prior washing or separation steps by adding silica beads, in particular magnetic silica beads, for absorbing RNA directly into the mixture obtained in step c).

Whether the cell lysing reagent is suitable for ommiting such a washing or separation step can be tested by (i) performing at least steps c) to e) of the method according to the present invention without any washing or separation step prior to step d) with at least two aliquots of an anti-coagulated whole blood sample and the cell lysing reagent to be tested on the one hand and a reference buffer such as the RNA/DNA Stabilization Reagent for Blood/Bone Marrow (manufacturer: Roche; Cat. No. 11 934 317 001) on the other hand, and (ii) comparing the amount and the quality of RNA detected in step e) and/or the Ct-values measeured in step e). The amount of RNA can e.g. be measured in spectrophotometric method using e.g. Nanodrop 1000. The quality of RNA can e.g. be verified in an electrophorese using e.g. the Experion system. If the cell lysing reagent to be tested results in the same or an equivalent amount and quality of RNA as the reference lysing reagent, then the tested cell lysing reagent is suitable for ommiting such a washing or separation step prior to step d). An equivalent amount and quality of RNA thereby refers to an amount and quality of RNA of at least about 80%, 85%, 90% or 95% or in a range of about 80% to about 150%, about 85% to about 140%, about 90% to about 130%, or about 95% to about 120% of the amount and quality of RNA obtained by the reference buffer. In addition or alternatively, if the Ct-values measured in step e) are basically the same, the cell lysing reagent is suitable for ommiting such a washing or separation step prior to step d). Basically the same means thereby that the Ct-value obtained with the cell lysing reagent to be tested is in a range of about 80% to about 150%, about 85% to about 140%, about 90% to about 130%, or about 95% to about 120% of the Ct-value obtained with the reference buffer.

A cell lysing reagent requiring an additional washing or separation step according to the tests performed by the inventors is RLT Puffer (QIAGEN - Cat No. 79216). Accordingly, in a preferred embodiment of the present invention the cell lysing reagent of step c) is not RLT Puffer (QIAGEN - Cat No. 79216) or a variant or equivalent thereof, which requires an additional washing or separation step.

In a preferred embodiment step c) of the method according to the present invention is not performed in PAXgene tubes as e.g. PAXgene Blood RNA tube (QIAGEN - Cat No. 762125) or an equivalent or variant thereof.

In a further preferred embodiment of the present invention step c) is not performed in an erythrocyte lysis buffer, e.g. comprising ammonium chloride or ammonium-chloride-potassium. Moreover, step c) is preferably not performed in EL (QIAGEN - Cat No. 79217) or any equivalents or variants of these buffers.

Step d) of the present invention is preferably performed by extracting RNA direclty from the mixture obtained in step c) without any prior washing or separation steps or a combination of separation and washing steps. In particular, step d) of the present invention is preferably performed by extracting RNA direclty from the mixture obtained in step c) without any centrifugation step prior to RNA extraction.

Step d) of the present invention is preferably performed by a solid phase extraction method using silica beads, in particular magnetic silica beads, for absorbing RNA. Especially preferred is an automated RNA extraction, in particular an automated RNA extraction using silica beads, in particular magnetic silica beads, for absorbing RNA. In a preferred embodiment the MagNA Pure-technology provided by the manufacturer Roche or an equivalent or variant thereof is used for extracting the RNA.

In a highly preferrred embodiment of the present invention step d) is performed by extracting RNA direclty from the mixture obtained in step c) without any prior washing or separation steps by a solid phase extraction method using magnetic silica beads for absorbing RNA. For doing this, the magnetic silica beads for absorbing RNA are directly added to the mixture of step c) without any prior washing or separation steps. The RNA is then extracted from the mixture by separating the added magnetic silica beads from the remaining mixture by using a magnet.

In a preferred embodiment of the invention a 1-step RT-qPCT is used for detecting the marker/s in step e). If RT-qPCT is used the gathered real-time PCR data (real-time PCR data) are preferably normalized by using a fixed reference value, which is not influenced by the conditions of the experiment, in order to achieve a precise gene expression quantification. For this purpose the expression of a reference gene is also measured in order to perform a relative comparison of amounts. The reference gene is preferably measured in the first and in the second aliquot. Preferred reference genes are 60S acidic ribosomal protein P0 (RPLPO), β-actin, glyceraldhyde-3-phosphate-dehydrogenase (GAPDH), porphobilinogen deaminase (PBGD), hypoxanthin-phosphoribosyl-transferase 1 (HPRT1); TATA box binding protein (TBP)-associated factor, RNA polymerase I, A (TAF1A) or tubulin.

The reference gene may also be called housekeeping gene. In a preferred embodiment of the present invention one or two housekeeping gene(s) is/are detected. In a further preferred embodiment of the present invention the at least one housekeeping gene in step e) is RPLPO.

In a particularly preferred embodiment of the present invention IFNG and RPLPO or IFNG, CXCL10 and RPLPO are detected in step e).

In a preferred embodiment of the present invention the RT-qPCR is performed by using at least two, more preferably at least three, dyes. Preferably, at least one dye is 6-Carboxyfluorescein (6-FAM) (Thermofisher Cat. No. C1360), hexachloro-fluoresceine (HEX) (Thermofisher, Cat. No. 403170) or Cyanine 5 monoacid (Cy5) (AAT Bioquest, Cat. No. 150). In a further preferred embodiment, the dyes FAM and HEX or the dyes 6-FAM, HEX and Cy5 are used. In particular the dyes FAM and HEX are used for detecting IFNG and RPLPO and the dyes FAM, HEX and Cy5 are used for detecting INFG, CXCL10 and RPLPO.

The RT-qPCR in step e) of the present invention may be performed by using a reference dye such as ROX or Mustang Purple. Such a reference dye is usually used to identify any pipetting errors or for checking the validity of an amplification. However, the inventors of the present invention have surprisingly found out that the sensitivity and specificity of the method according to the present invention can even be improved if the RT-qPCR is performed without ROX (carboxy-X-rhodamine). Thus, in a preferred embodiment the RT-qPCR is preferably performed without ROX.

In a further preferred embodiment of the method according to the present invention the marker IFNG and optionally CXCL10, is/are normalized to the housekeeping gene(s) detected in step e), preferably to RPLPO.

In a further preferred embodiment of the method according to the present invention step c) comprises additionally a step of adding a cell lysing reagent to a second aliquot of a sample of the individual, wherein the second aliquot has not been incubated with the at least one antigen. The second aliquot is preferably treated in the same way as the first aliquot with the only exception that no antigen is added in step a). For adapting the volume of the second aliquot to the volume of the stimulated first aliquot, water or a suitable buffer such as PBS may be added to the second aliquots. However, in a preferred embodiment the volume of the second aliquot is not adapted to the volume of the stimulated first aliquot. In step b) the second aliquot is preferably stored at the same conditions at which the first aliquot is incubated.

In a further preferred embodiment of the method according to the present invention step a) comprises additionally a step of contacting a third aliquot of a sample of an individual with a stimulation control. The stimulation control preferably stimulates cells to produce IFNG. A preferred stimulation control is Phytohemagglutinin (PHA), Lipopolysaccharide (LPS), a combination of anti-CD3 and anti-CD28 antibodies, Staphylococcal enterotoxin B (SEB) or a combination of PMA (Phorbol 12-myristate 13-acetate) and Ionomycin. Preferably, steps b), c), d) and e) are additionally performed for the third aliquot, wherein the third aliquot is incubated in step b) with the stimulation control.

In a further preferred embodiment of the present invention step e) is additionally performed for a positive control. The positive control is preferably a plasmid comprising fragments of the marker RNAs and the housekeeping gene(s) to be detected in step e), wherein said fragments comprise at least the nucleic acid sequences of the marker RNAs and the housekeeping gene(s) to which the corresponding primers and probes hybridize. The positive control is preferably used for controlling if the markers to be detected are detectable under the respective conditions of the RT-qPCR. Thus, the positive control comprises preferably only the positive control and the same reagents used for the detection of the markers to be detected in step e) which are added in step e) to the first, second and optional third aliquot. That means that the positive control does not comprise any RNA extracted in step d).

In a further preferred embodiment of the present invention steps d) and e) are additionally performed for a negative extraction control. The negative extraction control comprises preferably a mixture of PBS and the cell lysing reagent which is able to stabilize RNA as used in step c), preferably a mixture of 1: 1.25.

In a further preferred embodiment of the method according to the present invention, the method comprises additionally a step of adding an inhibition control to the first, second and/or optional third aliquot. Said inhibition control is preferably a natural or synthetic marker RNA, which is not normally found in the patient's sample. Example of suitable inhibition controls are bacteriophage MS2, isolated RNA of bacteriophage MS2, synthetically produced stabilized RNA fragments of the phage MS2 or any of the inbition controls listed in Baker et al., Nature Methods, vol .2 No.10, October 2005, pp. 731-734. The natural or synthetic marker RNA is preferably stabilized. The inhibition control is preferably used for controlling if the sample of the individual comprises any components which inhibit the detection of the marker genes to be detected in step e). Thus, the inhibition control may be added to the first, second, third and/or an optional further aliquot comprising the sample of the individual. Preferably it is added only to one of said aliquots. If the inhibition control is added to a further aliquot, steps a), b), c), d) and e) are also performed for said further aliquot, wherein step e) comprises additionally an step of detecting the inhibition control using a respective 1-step or 2-step reverse transcription quantitative real-time polymerase chain reaction (RT-qPCR) under the same conditions as performed for the other aliquots and optional controls.

Normally in a method of detection each value should be evaluated at least twofold, more preferably threefold, and a mean value of the evaluated value should be calculated for minimizing the risk of false positive or false negative values. The inventors of the present invention have surprinsingly found that the method of detection according to the present invention is very robust and it is sufficient to evaluate only one value from each sample, i.e. to contact only one first aliquot with the at last one antigen, to contact only one third aliquot with the stimulation control (in case a stimulation control is used) and to perform only one RT-qPCR run for the first, second and optionally third aliquot, the positive control and/or the negative extraction control. This is in particular true if the method according to the present invention comprises a stimulation control, a positive control and a negative control as described above.

In a further preferred embodiment step e) is performed by analysing a detectable change in marker expression in the first aliquot in comparison to the second aliquot, preferably above a certain treshhold. Alternatively, step e) may be performed by a classifyier analysis or classification method. Preferably, step e) of the method according to the present invention comprises (i) the comparison of the amount of the detected marker(s) of the first aliquot with the amount of the detected marker(s) of the second aliquot, (ii) a fold change analysis of the detected marker(s) in the first and in the second aliquot, or a combination of (i) and (ii). The comparison of the detected marker(s) in the first aliquot with the detected marker(s) in the second aliquot is preferably not performed by subtracting the detected marker(s) level in the second aliquot from the detected marker(s) level in the first aliquot. In fact, the comparison of the detected marker(s) is preferably performed by dividing the amount of marker in the first aliquot (the stimulated aliquot) by the amount of marker in the second aliquot (the unstimulated aliquot). Thus, an n-fold difference in amount of the marker of the first aliquot relative to the second aliquot is detected. Such an analysis is called fold change analysis.

In a preferred embodiment a difference in marker expression in the first and second aliquot is indicative that the individual is infected with pathogens causing tuberculosis or has been in contact with a pathogen causing tuberculosis. The difference in marker expression may be a detectable change in marker expression in the first aliquot in comparison to the second aliquot, preferably above a certain treshhold and/or may be determined by a classifyier analysis. Particularly preferred is a combination of fold change analysis and random forest analysis.

In a preferred embodiment the method according to the present invention comprises an additional step (g) of detecting an infection with pathogens causing tuberculosis and/or differentiating individuals being infected with pathogens causing tuberculosis and individuals being uninfected with pathogens causing tuberculosis based on the comparison performed in step (f). Said additional step (g) may comprise the step of determining whether the individual is infected with pathogens causing tuberculosis or has been in contact with pathogens causing tuberculosis. In particular, step (g) may comprise the indication whether it is likely that the individual of which the sample was obtained is infected with pathogens causing tuberculosis or has been in contact with a pathogen causing tuberculosis. Preferably, step (g) may comprise calculating the probability that the person from which the sample was obtained is infected with pathogens causing tuberculosis or has been in contact with pathogen causing tuberculosis. Alternatively or in addition, step (g) may comprise the calculation of the probability that the person from which the the sample was obtained is not infected with pathogens causing tuberculosis or has not been in contact with pathogen causing tuberculosis. Step (g) can be performed subsequent to step (f) or may be incorporated into step (f).

If the fold change of IFNg in the first and second aliquod is below about 1.68,. more preferably is about 1.62 this is an indication that the sample is of an individual having no infection with pathogens causing tuberculosis. If the fold change of IFNg in the first and second aliquod is above about 3.36, more preferably is about 2.14 this is an indication that the sample is of an individual having an infection with pathogens causing tuberculosis. If the fold change of CXCL10 in the first and second aliquod is below about 8 , more preferably is about 4.92 this is an indication that the sample is of an individual having no infection with pathogens causing tuberculosis. If the fold change of CXCL10 in the first and second aliquod is above about 38, more preferably is about 27.86 this is an indication that the sample is of an individual having an infection with pathogens causing tuberculosis.

Step f) and optionally (g) may be performed by a classification method as e.g. artificial neural networks, logistic regression, decision trees, Random Forest, Least Absolute Shrinkage and Selection Operator (LASSO), support vector machines (SVMs), threshold analysis, linear discriminant analysis, k-Nearest Neighbor (kNN), Naive Bayes, Bayesian Network, or any other method developing classification models known in the art.

In a preferred embodiment a Random Forest approach is performed as the classification method. Random Forests (Breiman 2001. "Random Forests". Machine Learning. 45: 5-32; doi:10.1023/A:1010933404324) are an ensemble learning method for classification, regression and other tasks, that operate by constructing a multitude of decision trees at training time and outputting the class that is the mode of the classes (classification) or mean prediction (regression) of the individual trees. Random Forests correct for decision trees' habit of overfitting to their training set.

The random Forest approach can be performed by a basic Random Forest approach or by a probability Forest approach. The basic Random Forest approach denotes the original Random Forest implementation by Leo Breiman (2001, Machine Learning. 45 (1): 5-32; doi:10.1023/A:1010933404324) and the package ranger software may be used to perform this kind of Random Forest training and application. The probability Forest approach is based on the implementation of Random Forest proposed by Malley et al. (2012, Methods Inf Med 51:74-81; http://dx.doi.org/10.3414/ ME00-01-0052) for probability estimation. The package ranger may be used to perform probability Forest training and application.

In order to get smoother probability estimations, the probability Forests were parametrized as follows: number of trees = 1e3, minimal node size = 5, split rule = "extratrees" with number of random split set to 5, and number of variables to possibly split at in each node set to 1. Generating classifiers with smoother probability estimations has also the aim to generate classifiers boundaries that will be more similar to those that would have been generated by a human process and limit overfitting. This corresponds to the following parameter setting in package ranger: number of trees (num.trees) = 1e3, minimal node size (min.node.size) = 5, split rule = "extratrees", with the number of random splits (num.random.splits) set to 5 and the number of variables to possibly split at (mtry) set to 1. The use of Extra Trees (Geurts et al., 2006, Machine Learning. 63: 3-42; doi:10.1007/s10994-006-6226-1) is essentially motivated by the fact that resulting models are thus smoother than the piecewise constant ones obtained with other random forest implementations.

Practically, Random Forest classifiers may be established by using the software R [3.5.0] in combination with the packages ranger [0.9.0], readxl [1.1.0], stringr [1.3.0] and mlr [2.12.1]. The measurements of samples (as fold-change of antigen stimulation) were log2-transformed before training using the function *ranger()*, with the parameters described above.

In a particularly preferred embodiment of the present invention a combination of fold change analysis and random forest analysis is performed.

If the difference in marker expression in the first and second aliquot is indicative that the individual is infected with pathogens causing tuberculosis, the method according to the present invention may further comprise a step of administering a treatment to said individual. Preferably, said treatment comprises administering to the individual an amount of a therapeutic agent or a combination of therapeutic agents effective to treat tuberculosis. As needed, said therapeutic agent or combination of therapeutic agents is preferably effective to treat active tuberculosis or latent infection with pathogens causing tuberculosis or both.

Thus, a further aspect of the disclosure which is not part of the invention refers to a method of detecting an infection with pathogens causing tuberculosis and/or a method of treating and/or preventing tuberculosis, said method comprises:

(a) contacting a first aliquot of a sample of an individual with at least one antigen of a pathogen causing tuberculosis, in particular wherein a heterodimer of two antigens, more preferably a heterodimer of ESAT-6 and CFP-10, is added to the first aliquot or wherein the first aliquot is comprised in a container comprising a heterodimer,
b) incubating the first aliquot with the at least one antigen for about 15 to about 23 hours,
c) adding a cell lysing reagent which is preferably able to stabilize RNA in a temperature range of about 0°C to about 40°C, more preferably of about 2°C to about 30°C, or of about 15°C to about 30°C, or of about 2°C to about 8°C, or of about 15°C to about 25°C, or about of 18°C to about 30°C for about 2 h to about 48 h, more preferably for about 12 h to about 48 h more preferably for about 2 h to about 72h, more preferably for about 12 h to about 72h,
d) performing an RNA extraction from the mixture obtained in step c), preferably an automated RNA extraction, wherein RNA is extracted from the mixture obtained in step c) without any prior washing or separation steps,
e) detecting in the first aliquot and in a second aliquot of the sample of the individual at least the marker IFNG and at least one housekeeping gene(s), more preferably at least the markers IFNG and CXCL10 and at least one housekeeping gene using a 1-step or 2-step reverse transcription quantitative real-time polymerase chain reaction (RT-qPCR), wherein the second aliquot has not been incubated with the at least one antigen,
f) comparing the detected markers in the first aliquot with the detected markers in the second aliquot in a fold change analysis, more preferably in a classifier method,
g) evaluating whether the difference in marker expression in the first and second aliquot is indicative that the individual is infected with pathogens causing tuberculosis, and

h) administering an effective amount of a therapeutic agent or a combination of therapeutic agents effective to treat tuberculosis to the individual evaluated to be infected with pathogens causing tuberculosis.

The evaluation whether the difference in marker expression in the first and second aliquot is indicative that the individual is infected with pathogens causing tuberculosis may be performed by detecting an infection with pathogens causing tuberculosis in accordance with the present invention as described above.

In a further embodiment the present invention refers to a method of treating and/or preventing tuberculosis, said method comprises: administering an effective amount of a therapeutic agent or a combination of therapeutic agents effective to treat tuberculosis to an individual diagnosed to be infected with pathogens causing tuberculosis, wherein the respectively diagnosed individual has been diagnosed by the method according to the present invention as described herein. Before said individual is treated in accordance with the present invention said individual may be diagnosed in a second subsequent diagnosis step (i) to have a latent infection with pathogens causing tuberculosis, (ii) to suffer from an active tuberculosis infection or (iii) to have been in contact with pathogens causing tuberculosis, wherein the pathogens have successfully been killed or combated. Said second subsequent diagnosis step may be performed as known in the art and described herein.

Therapeutic agent(s) effective to treat and/or prevent tuberculosis may comprise therapeutic agents which are effective to kill, eliminate and/or neutralize pathogens causing tuberculosis and/or therapeutic agents which are effective in supporting the immune system of the individual to kill, eliminate and/or neutralize pathogens causing tuberculosis. Examples for suitable therapeutic agents are Rifapentine (RPT), Rifampin (RIF), Isoniazid (INH), Ethambutol (EMB) and Pyrazinamide (PZA), Rifabutin, Pyrazinamide, Ethambutol, Cycloserine, Ethionamide, Streptomycin, Amikacin/kanamycin, Capreomycin, Para-amino salicylic acid, Levofloxacin and Moxifloxacin. Said therapeutic agents may be administered alone or in combination with each other or in combination with further suitable therapeutic agents. In particular, a combination of Isoniazid and Rifapentine or a combination of Isoniazid, Rifampin, Pyrazinamide and Ethambutol is preferred.

If the difference in marker expression in the first and second aliquot is indicative that the individual is infected with pathogens causing tuberculosis, the method according to the present invention may comprise prior to the treating step a step of performing a differential diagnosis. Said differential diagnosis comprises preferably the step of determining whether the infected individual suffers from a latent infection with pathogens causing tuberculosis, an active tuberculosis, or has been in contact with pathogens causing tuberculosis, wherein the pathogens have successfully been killed or combated. Said differential diagnosis may for example be performed as described in the following publications: Lewinsohn et al. "Official American Thoracic Society/Infectious Diseases Society of America/Centers for Disease Control and Prevention Clinical Practice Guidelines: Diagnosis of Tuberculosis in Adults and Children", CID 2016;00(0):1-33; "Bericht zur Epidemiologie der Tuberkulose in Deutschland für 2016" provided by Robert Koch Institut; and Seybold, Ulrich, "Latente Tuberkulose - Infektion und Immunschwäche", HIV&more 2/2016.

Individuals with a latent infection with pathogens causing tuberculosis usually do not have symptoms and they cannot spread tuberculosis bacteria to other. However, there is a risk that latent tuberculosis bacteria become active in the body and multiply. Thus, individuals having such a latent infection may for example be treated by the following Latent TB Infection Treatment Regimens published by the Centers for Disease Control and Prevention (CDC):

| Drugs | Duration Interval |
| --- | --- |
| Isoniazid and Rifapentine | 3 months Once weekly |
| Rifampin | 4 months Daily |
| Isoniazid | 6 months Daily or twice weekly |
| Isoniazid | 9 months Daily or twice weekly |

When TB bacteria become active (multiplying in the body) and the immune system is not able to stop the bacteria from growing, this is called TB (tuberculosis) disease or active tuberculosis. Individuals having active tuberculosis may for example be treated by the following TB Infection Treatment Regimens published by the Centers for Disease Control and Prevention (CDC):

| | | INTENSIVE PHASE | | CONTINUATION PHASE | | |
|---|---|---|---|---|---|---|
| Regimen | Drugs | Interval and Dose (minimum duration) | Drugs | Interval and Dose (minimum duration) | Range of Total Doses [mg] |
| 1 | INH RIF PZA EMB | 7 days/week for 56 doses (8 weeks) *or* 5 days/week for 40 doses (8 weeks) | INH RIF | 7 days/week for 126 doses (18 weeks) *or* 5 days/week for 90 doses (18 weeks) | 182 to 130 |
| 2 | INH RIF PZA EMB | 7 days/week for 56 doses (8 weeks) *or* 5 days/week for 40 doses (8 weeks) | INH RIF | 3 times weekly for 54 doses (18 weeks) | 110 to 94 |
| 3 | INH RIF PZA EMB | 3 times weekly for 24 doses (8 weeks) | INH RIF | 3 times weekly for 54 doses (18 weeks) | 78 |
| 4 | INH RIF PZA EMB | 7 days/week for 14 doses then twice weekly for 12 doses | INH RIF | Twice weekly for 36 doses (18 weeks) | 62 |

Alternatively, individuals may be treated by tuberculosis treatment methods known in the art as e.g. described in Nahid et al. ("Official American Thoracic Society/Centers for Disease Control and Prevention/Infectious Diseases Society of America Clinical Practice Guidelines: Treatment of Drug-Susceptible Tuberculosis", ATS/TS/CDC/IDSA Clinical Practice Guidelines for Drug-Susceptible TB • CID 2016:63 (1 October), e147-e195).

The marker IFN-$\gamma$ is well known in the art and is e.g. secreted by specifically restimulated antigen-specific memory T cells, in particular Th-1 cells and cytotoxic T cells. Multiple variants of IFN-$\gamma$ are known in the art. Preferably, the marker IFN-$\gamma$ is human IFN-$\gamma$. In one embodiment of the present invention the marker IFN-$\gamma$ is encoded by a nucleic acid molecule comprising a nucleic acid sequence according to SEQ ID NO:1 or a functional variant thereof. Preferably, a IFN-$\gamma$ functional variant may comprise a nucleic acid sequence having at least 60%, more preferably 70%, 80% or 90% sequence identity with the sequence of SEQ ID NO: 1. Preferably, a functional variant is a variant which expression is altered if the method according to the present invention is performed with a sample obtained from an individual having acute tuberculosis. The alteration of expression is preferably above a certain threshold. preferably above 1.1. The term "IFN-y" may be used interchangeable with the terms "INF-g", "INFG", "INF-gamma" and "INF-□", IFN-g", "IFNG", "IFN-gamma" and "IFN-□.

In RT-qPCR any suitable primer that specifically binds to nucleic acids of IFN-$\gamma$ may be used for detecting IFN-$\gamma$. Examples for suitable primers are nucleotides comprising a nucleic acid sequence according to SEQ ID NO: 2 and 3. Preferably, in addition to the primers a probe that specifically binds to nucleic acids of IFN-$\gamma$ is used. For example, a nucleic acid sequence comprising a sequence according to SEQ ID NO: 4 may be used as a probe. Said probe may comprise a fluorescence dye such as 6-Carboxyfluorescein (6-FAM) (Thermofisher Cat. No. C1360) and/or quencher such as Tide Quencher™ 2 acid (TQ2) (AAT Bioquest, Cat. No. 2200).

The marker CXCL-10 is also known as IP-10 and is a small chemokine expressed by APCs and a main driver of proinflammatory immune responses. CXCL-10 is expressed by cells infected with viruses and bacteria, but can also be induced at high levels as part of the adaptive immune response. In this case, CXCL-10 secretion is initiated when T cells recognize their specific peptide presented on the APC. IP-10 secretion appears to be driven by multiple signals, mainly T-cell-derived IFN-g, but also IL-2, IFN-a, IFN-b, IL-27, IL-17, IL-23, and autocrine APC-derived TNF and IL-1b. Multiple variants of CXCL-10 are known in the art. Preferably, the marker CXCL-10 is human CXCL-10. In one embodiment of the present invention the marker CXCL-10 is encoded by a nucleic acid molecule comprising a nucleic acid sequence according to SEQ ID NO: 5 or a functional variant thereof. Preferably, a CXCL-10 functional variant may comprise a nucleic acid sequence having at least 60%, more preferably 70%, 80% or 90% sequence identity with the sequence of SEQ ID NO: 5. Preferably, a functional variant is a variant which expression is altered if the method according to the present invention is performed with a sample obtained from an individual having acute tuberculosis. The alteration of expression is preferably above a certain threshold, more preferably above 1.1.

In RT-qPCR any suitable primer that specifically binds to nucleic acids of CXCL-10 may be used for detecting CXCL-10. Examples for suitable primers are nucleotides comprising a nucleic acid sequence according to SEQ ID NO: 6 and 7. Preferably, in addition to the primers a probe that specifically binds to nucleic acids of CXCL-10 is used. For example, a nucleic acid sequence comprising a sequence according to SEQ ID NO: 8 may be used as a probe. Said probe may comprise a fluorescence dye such as Cy5 and/or quencher suach as TQ3.

The housekeeping gene RPLPO refers to the 60S acidic ribosomal protein P0. In one embodiment of the present invention the RPLPO is encoded by a nucleic acid molecule comprising a nucleic acid sequence according to SEQ ID NO: 9. or a functional variant thereof. In RT-qPCR any suitable primer that specifically binds to nucleic acids of RPLPO may be used for detecting RPLPO. Examples for suitable primers are nucleotides comprising a nucleic acid sequence according to SEQ ID NO: 10 and 11. Preferably, in addition to the primers a probe that specifically binds to nucleic acids of RPLPO is used. For example, a nucleic acid sequence comprising a sequence according to SEQ ID NO: 12 may be used as a probe. Said probe may comprise a fluorescence dye such as HEX and/or quencher such as Tide Quencher™ 2 acid (TQ2) (AAT Bioquest, Cat. No. 2200).

The present invention also provides a kit for performing a method according to the present invention or one or more single steps thereof, which kit is defined in the claims Preferably said kit comprises additionally at least two primer pairs primer and probes for detecting IFNG, optionally CXCL10, and at least one housekeeping gene, wherein each probe comprises preferably a dye and a quencher. The primer pairs primer and probes for detecting IFNG, optionally CXCL10, and at least one housekeeping gene may however also be provided in a second kit as defined ion the claims.

In a preferred embodiment of the present invention the kit comprises

- a heterodimer of ESAT-6 and CFP-10,
- a stimulation control,
- a cell lysing reagent which is able to stabilize RNA, wherein the cell lysing reagent is preferably provided in a light-protected packaging (e.g. sleeve),
- a reaction mixture comprising desoxynucleotides (dATP, dGTP, dCTP, dTTP) and a polymerase buffer solution, as e.g. NxtScript DNA Master (manufacturer: Roche; Ident-No. 07 368 143; Cat. No. 7368143103),
- a mixture comprising primer and probes for detecting IFNG and CXCL10, and at least one housekeeping gene, wherein each probe comprises preferably a dye and a quencher,
- a Reverse Transcriptase as e.g. NxtScript RT (manufacturer: Roche: Ident-No. 07 371 527; Cat. No. 7371527103),
- a positive control, in particular wherein the positive control is a plasmid comprising fragments of the marker RNAs and the housekeeping gene(s) to be detected in step e), wherein said fragments comprise at least the nucleic acid sequences of the marker RNAs and the housekeeping gene(s) to which the corresponding primers and probes hybridize, and
- a negative extraction control, in particular wherein the negative extraction control comprises a mixture of PBS and the cell lysing reagent which is able to stabilize RNA as used in step c), preferably a mixture of 1:1.25, wherein the cell lysing reagent which is able to stabilize RNA comprises a detergens such as such as Triton X 100, Tween 20, Tween 80, 3 [(3 Cholamidopropyl)dimethylammonio] 1 propanesulfonate (CHAPS), urea and/or n-Dodecyl-β- D-Maltopyranoside (DDM), a chaotropic agent such as a guanidinium lysis buffers comprising preferably guanidinium isothiocyanate and/or guanidinium-HCl and an antioxidant such as DTT, Dithioerythritol (DTE), Tris(2-carboxyethyl)phosphin (TCEP) and/or β- Mercaptoethanolopic agent.

Since steps a), b) and c) may be performed at a different place than steps d) and e), the present invention also provides a kit for performing single steps of the method according to the present invention. In particular, the present invention refers to a first kit for particularly performing steps a), b) and c) of the method according to the present invention. Said first kit comprises

- a heterodimer of ESAT-6 and CFP-10,
- a stimulation control, and
- a cell lysing reagent which is able to stabilize RNA, wherein the cell lysing reagent is preferably provided in a light-protected packaging (e.g. sleeve).

The heterodimer of ESAT-6 and CFP-10 may be coated on the inner surface of a blood sampling tube (e.g. a Heparin tube). The heterodimer may be released from the inner surface into the aliquot e.g. by slight shaking of the tube.

In addition, the present invention refers to a second kit for particularly performing step e) of the method according to the present invention. Said second kit comprises

- a reaction mixture comprising desoxynucleotides (dATP, dGTP, dCTP, dTTP) and a polymerase buffer solution, as e.g. NxtScript DNA Master (manufacturer: Roche; Ident-No. 07 368 143; Cat. No. 7368143103),
- a mixture comprising primer and probes for detecting IFNG and CXCL10, and at least one housekeeping gene, wherein each probe comprises preferably a dye and a quencher,
- a Reverse Transcriptase as e.g. NxtScript RT (manufacturer: Roche: Ident-No. 07 371 527; Cat. No. 7371527103),
- a positive control, in particular wherein the positive control is a plasmid comprising fragments of the marker RNAs and the housekeeping gene(s) to be detected in step e), wherein said fragments comprise at least the nucleic acid sequences of the marker RNAs and the housekeeping gene(s) to which the corresponding primers and probes hybridize, and
- a negative extraction control, in particular wherein the negative extraction control comprises a mixture of PBS and the cell lysing reagent which is able to stabilize RNA as used in step c), wherein the cell lysing reagent which is able to stabilize

RNA comprises a detergens such as such as Triton X 100, Tween 20, Tween 80, 3 [(3 Cholamidopropyl) dimethylammonio] 1 propanesulfonate (CHAPS), urea and/or n-Dodecyl-$\beta$- D-Maltopyranoside (DDM), a chaotropic agent such as a guanidinium lysis buffers comprising preferably guanidinium isothiocyanate and/or guanidinium-HCl and an antioxidant such as DTT, Dithioerythritol (DTE), Tris(2-carboxyethyl)phosphin (TCEP) and/or $\beta$- Mercaptoethanolopic agent.

The negative extraction control comprises preferably a mixture of PBS and the cell lysing reagent of about 1:1 to about 1:1.5, more preferably of about 1:1.25.

The cell lysing reagent of the negative extraction control of the second kit is preferably the same as the cell lysing reagent of the first kit.

The reaction mixture and the mixture comprising primer and probes of the kits according to the present invention may be combined in one master mixture.

Since the kits provided by the present invention are for performing the method according to the present invention, all embodiments of the required components needed for performing the method according to the present invention apply to the kit components as outlined above.

In the following the invention is illustrated by the subsequent examples. These examples are to be considered as specific embodiments of the invention and shall not be considered to be limiting.

### Example 1: Detection of infection with pathogens causing tuberculosis from whole blood samples of patients with latent *M. tuberculosis* infection and active tuberculosis

The aim of this pilot study was to assess the suitability of an optimized RT-qPCR-based work-flow for a reliable and practicable identification of individuals infected with tuberculosis pathogens. In this experiment anticoagulated whole blood samples of 28 patients latently infected with tuberculosis pathogens, 37 patients with active tuberculosis and for control 42 healthy donors without known contact with tuberculosis pathogens were tested.

### Sample preparation and stimulation

At least 3 ml whole blood was drawn from each study participant using sodium heparin collection tubes and maintained at room temperature (18 - 25°C) for a maximum of 8 hours prior to processing. The following steps were performed under sterile conditions in a class II biosafety laminar flow cabinet.

Before aliquoting, the collection tube was mixed briefly and gently by inverting. Then each one milliliter (1 ml) heparinized blood was transferred into two 5 ml polypropylene round-bottom stimulation tubes. Aliquots were stimulated with 20 $\mu$g/ml of a heterodimer of *M. tuberculosis* proteins ESAT6 and CFP10 (1 mg/ml ESAT6/CFP-10 complex, K10111; Lophius Biosciences GmbH) (Tab. 1). An unstimulated tube served as negative control. After addition of stimulants, tubes were loosely closed (i.e. the caps were only loosely on the tube (first pressure point)), mixed gently by flicking the tubes several times and then incubated overnight (15 to 23 h) at 37°C in a humified 5% $CO_2$ incubator.

Following stimulation, RNA was stabilized by addition of 1.25 ml SRR stabilization reagent (Roche Art. No. 11934317001) to each of the 2 tubes. Tubes were tightly closed and mixed vigorously for 5 to 10 sec. by vortexing. Prior to first use, the RNA stabilizer was prewarmed at 37°C for 30 min, mixed and checked for the absence of precipitates. Stabilized samples were directly processed for RNA extraction or quickly frozen using dry ice and stored below -70°C until further processing.

**Table 1: Sequence information for stimulants**

| Antigen | Gene | Expasy ID | Size | Sequence |
|---------|------|-----------|------|----------|
| ESAT-6 | esxA | ESXA_MYCTO | 102 AA 10867.89 Da | MEQQWNFAGIEAAASAIQGNVTSIHSLLDEGKQSL TKLAAAWGGSGSEAYQGVQQKWDATATELNNAL QNLARTISEAGQAMASTEGNVTGMFA – LEHHHH HH (SEQ ID NO: 13) |
| CFP-10 | esxB | ESXB_MYCTU | 111 AA 12072.16 Da | MAEMKTDAATLAQEAGNFERISGDLKTQIDQVEST AGSLQGQWRGAAGTAAQAAVVRFQEAANKQKQE LDEISTNIRQAGVQYSRADEEQQQALSSQMGF – AAALEHHHHHH (SEQ ID NO: 14) |

**RNA extraction**

Then, stabilized RNA was extracted from stimulation tubes using a MagNA Pure 96 instrument (Roche - Cat No. 06541089001) and the "MagNA Pure 96 Cellular RNA Large Volume Kit" (Roche - Cat No. 05467535001).

In case of RNA extraction directly after blood sample stabilization, samples were briefly vortexed and transferred to the 'MagNA Pure 96 Processing Cartridge' (Roche 06241603001). - In case stabilized samples are stored frozen until RNA extraction, samples were thawed completely, vortexed and transferred to the 'MagNA Pure 96 Processing Cartridge'.

Either 900µl or 1800µl SRR-stabilized lysate were transferred into one well of a MagNA Pure 96 Processing Cartridge and the predefined "RNA Blood LV 400" or "RNA Blood LV 800" MagNA Pure 96 protocols were run, respectively. Samples were eluted in 50µl or 100µl of the kit's elution buffer for the " RNA Blood LV 400" or " RNA Blood LV 800" protocols, respectively.

**cDNA synthesis and qPCR**

After extraction, RNA samples were directly tested by RT-qPCR. Thereby, RNA samples were kept on ice during the entire procedure. Alternatively, to direct testing by RT-qPCR, extracted RNA can be stored below -70°C until further testing.

**RT-qPCR Reaction**

PCR measurements of samples were performed in triplicates using a 96-well reaction plate setup. First the Master Mix was prepared by adding the components listed in table 2 and vortexing the mix 3x for one second. 18 µl of the RT-qPCR Master Mix were pipetted per well of an optical 96-well reaction plate. Then, 2 µl of template were added the following order: extracted RNA samples (two per donor: unstimulated and TB antigen-stimulated, each in three replica). At that step extreme care was taken that no air bubbles are generated during pipetting of reagents into the 96-well plate, as they can interfere with the fluorescence reading during the RT-qPCR run. Then, plates were carefully and tightly closed with an adhesive optical foil seal. Then the plate was consistently and thoroughly vortexed for 10 seconds and centrifuged for approximately 1 min at 177 x g and proceeded to the setup of the real-time PCR instrument. The setup 96-well plate was stored at +2-8°C in the dark for up to 10 min.

**Table 2: Preparation of the Master Mix**

| | final conc. | Per well (µl) |
|---|---|---|
| NxtScript DNA Master (5x), Roche, Cat. No.: 07368143103 | 1x | 4 |
| NxtScript RT-Enzym (85U/µl), Roche, Cat. No.: 07371527103 | 1x | 0,1 |
| H$_2$O (PCR-grade), Roche, Cat. No.: 03036430103 | | 8,82 |
| F-Primer (10 µM): 0208ra | 178 nM | 0,356 |
| R-Primer (10 µM): 0209ra | 178 nM | 0,356 |
| probe (10 µM): S0095ra | 300 nM | 0,6 |
| F-Primer (10 µM): 0204ia | 400 nM | 0,8 |

(continued)

|  | final conc. | Per well (μl) |
|---|---|---|
| R-Primer (10 μM): 0205ia | 400 nM | 0,8 |
| probe (10 μM): S0093ia | 300 nM | 0,6 |
| F-Primer (10 μM): 0206cc<br>R-Primer (10 μM): 0207cc | 267 nM<br>267 nM | 0,534<br>0,534 |
| probe (10 μM): 50094cc | 250 nM | 0,5 |
| Total volume with 2 μl template: | | 20 |

For detection of indicated makers following primers / probes or commercial assays have been used:

**Table 3: Sequence Information for Primer for qPCR**

| primer name | designation | gene HGNC name | sequence 5'-3' | amplicon length |
|---|---|---|---|---|
| 0204ia-F | IFNG_F | IFNG | AGATGACTTCGAAAAGCTGAC (SEQ ID NO:2) | 101 bp |
| 0205ia-R | IFNG_R | IFNG | GGCGACAGTTCAGCCATCA (SEQ ID NO: 3) | 101 bp |
| 0206cc-F | CXCL10_F | CXCL10 (IP-10) | ACTCTAAGTGGCATTCAAGGAGT (SEQ ID NO: 6) | 92 bp |
| 0207cc-R | CXCL10_R | CXCL10 (IP-10) | AAAGACCTTGGATTAACAGGTTGA (SEQ ID NO: 7) | 92 bp |
| 0208ra-F | RPLP0_F | RPLPO (huP0) | GAATGTGGGCTTTGTGTTCAC (SEQ ID NO: 10) | 113 bp |
| 0209ra-R | RPLP0_R | RPLPO (huP0) | TGACTTCACATGGGGCAATG (SEQ ID NO: 11) | 113 bp |

**Table 4: Sequence Information for Probes for qPCR**

| probe Name | dye / quencher | designation | gen HGNC name | accession Number | sequence 5'-3' |
|---|---|---|---|---|---|
| S0093ia | FAM / TQ2 | FAM-IFNG-TQ2 | IFNG | NM_000619.3 | FAM-TTGAATGTCCAACGCA AAGCAATACATGAA-TQ2 (FAM-SEQ ID NO: 4-TQ2) |
| S0094cc | Cy5 / TQ3 | Cy5-CXCL10-TQ3 | CXCL10 (IP-10) | NM_001565.3 | Cy5-CCTCTCTCTAGAACTGTA CGCTGTACCTGC-TQ3 (Cy5-SEQ ID NO: 8-TQ3) |
| S0095ra | HEX / TQ2 | HEX-RPLP0-TQ2 | RPLPO (huP0) | NM_001002.3 | HEX-GGTGCCAGCTGCTGCCCG TG-TQ2 (HEX-SEQ ID NO: 12-TQ2) |

The RT-qPCR was run on a QuantStudio 5 (Thermo Fisher - Cat No. A28138) Real-Time PCR system. The one-step RT-qPCR-protocol starts with an initial step for cDNA synthesis at 50°C for 10 min following an 95°C denaturation step for 30sec and then completes 40 cycles of 95°C for 5sec and subsequent 60° for 30sec with data collection during the latter.

DeltaRn values of 25000, 40000 and 80000 were used as thresholds for RPLPO, IFNG and CXCL10 Ct determination, respectively.

**Data analysis and fold change calculations**

For data analysis Ct mean values for replicates of marker genes and RPLPO samples were used. The DNA quantity (D) of marker genes and RPLPO was calculated using the Ct values (Ct) and the PCR efficiency (e) of each PCR reaction, using the following formula:

$$D = e^{-Ct}$$

Normalized DNA quantity for marker genes ($N_m$) was calculated using the DNA quantity of marker genes ($D_m$) and the DNA quantity of the housekeeping gene RPLPO ($D_n$) in the same samples, using the following formula:

$$N_m = D_m/D_h$$

For expression fold change calculations of each marker gene ($fc_m$) through stimulation the normalized DNA quantities from the stimulated ($N_m(S)$) and the unstimulated ($N_m(U)$) samples from each donor were used in the following formula:

$$fc_m = (N_m(S))/(N_m(U))$$

Fold change values were used to classify donors as TB-infected or -uninfected using a logistic regression classifier.

In this pilot experiment the optimized work-flow for the detection of infection with tuberculosis causing pathogens showed high sensitivity and specificity of 95,24 and 95,38%, respectively.

The accuracy of the test results was 95,33%. The performance parameters of the test are shown in tab. 5.

Tab. 5: Performance parameters of the infection detection test based on the optimized work-flow in a test collective of 1

|  |  | cases |
|---|---|---|
| Specificity [%] | 95,24 | (40 of 42 correct) |
| Sensitivity [%] | 95,38 | (62 of 65 correct) |
| Accuracy [%] | 95,33 | (102 of 107 correct) |
| latent recall [%] | 92,86 | (26 of 28 correct) |
| active recall [%] | 97,30 | (36 of 37 correct) |

**Example 2: Detection of infection with pathogens causing tuberculosis from whole blood samples of patients with latent *M. tuberculosis* infection and active tuberculosis using RT-qPCR triplicates compared with single RT-qPCR measurements**

The aim of this study was to examinate if the detection of infection with pathogens causing tuberculosis from whole blood samples of patients with latent *M. tuberculosis* infection and active tuberculosis would lead to reliable and practical results when using single RT-qPCR measurement instead of RT-qPCR triplicates.

For this study anticoagulated whole blood samples of 31 patients latently infected with tuberculosis pathogens, 61 patients with active tuberculosis and for control 83 healthy donors without known contact with tuberculosis pathogens were tested.

The sample preparation and stimulation was performed as described in example 1 above. Further, the RNA extraction, cDNA synthesis, RT-PCR and RT-qPCR were performed as described above in example 1.

Data analysis and fold change calculations were also performed as described above in example 1.

In contrast to the final assay design (i.e. 1 well per sample), all RT-qPCR runs contained technical triplicates during phase 2 a development. Using the replicates' mean Ct values for the data evaluation, the following performance characteristics for the detection of *M. tuberculosis* infection are obtained (table 6):

Tab. 6: Performance characteristics using the triplicate mean values

|  | Value (%) | 95% Confidence Interval (CI) (%) |
|---|---|---|
| **Specificity** | 94.81 | 87.00 - 98.36 |
| **Sensitivity** | 92.22 | 84.56 - 96.43 |
| **"Active Recall"** | 93.22 | 83.36 - 97.80 |
| **Invalid Rate** | 4.57 | - |

From development phase 3 on, the assay was only performed with a single RT-qPCR well per sample. To simulate this, an additional data evaluation was done: by combining the three wells of unstimulated with the three wells of TB antigen stimulated samples, 9 different data pairs are obtained that lead to slightly different FC values. During a random selection approach, one of these 9 single well combinations was picked for every donor. This random selection was repeated 500 times for the complete donor set. The table below gives the mean and median values for the performance characteristics for the detection of *M. tuberculosis* complex infection across the 500 random selections (table 7):

Tab. 7: Performance characteristics for the simulation of single well analysis

|  | Mean Value in % | Median Value in % |
|---|---|---|
| **Specificity** | 94.14 | 94.59 |
| **Sensitivity** | 92.15 | 92.31 |
| **"Active Recall"** | 92.76 | 93.22 |
| **Invalid Rate** | 4.11 | 4.00 |

Finally, the performance characteristics were determined by using only the first well of the RT-qPCR triplicates as an alternative way to simulate the results of a single well assay (table 8). The comparison to tables 6 and 7 reveals that the data derived from the 1st well result in slightly lower performance than obtained by the mean of triplicate measurements. This is considered to be a random effect. Furthermore, all obtained data (i.e. with the triplicate measurements as well as with the two approaches to simulate use of single wells only) result in assay performance values meeting the acceptance criteria.

Tab. 8: Performance characteristics using the 1st well of the triplicates

|  | Value (%) | 95% Confidence Interval (CI) (%) |
|---|---|---|
| **Specificity** | 93.15 | 84.59 - 97.39 |
| **Sensitivity** | 90.11 | 82.06 - 94.91 |
| **"Active Recall"** | 90.00 | 79.51 - 95.68 |
| **Invalid Rate** | 6.29 | - |

Even though the minimally required number of 40 measurements per group could be included in the analysis, the case numbers lead to relatively broad 95% confidence intervals (see tables 6 and 8). However, clinical performance is a key aspect of development phase 3 and will be investigated with higher case numbers during the respective performance study.

For this study, the sensitivity and specificity of the assay range above 90% both for the technical triplicates' mean (92.22% sensitivity, 94.81% specificity) and the simulation of single-well measurements (92.15% sensitivity, 94.14% specificity (mean of 500 random picks); (90.11% sensitivity, 93.15% specificity (1st well)).

**Example 3: Detection of infection with pathogens causing tuberculosis from whole blood samples of patients with active tuberculosis and uninfected patients using the markers IFNG and CXCL10 alone or in combination IFNG/CXCL10**

The aim of this study was to assess the suitability of an optimized RT-qPCR-based work-flow for a reliable and practicable identification of individuals infected with tuberculosis pathogens using single markers IFNG or CXCL10 or the combination of the two markers IFNG and CXCL10. In this experiment anticoagulated whole blood samples of 36 patients with active tuberculosis and for control 34 healthy donors without known contact with tuberculosis pathogens were tested.

The sample preparation and stimulation were performed as described in example 1 above. Further, the RNA extraction, cDNA synthesis, RT-PCR and RT-qPCR were performed as described above in example 1 with the exception that the RT-qPCR measurement is performed as a single well assay.

Data analysis and fold change calculations were also performed as described above in example 1 for the assays performed with the combination of the markers IFNG and CXCL10. In case of the single markers IFNG and CXCL10 used alone a threshold analysis was performed. The fold change threshold for CXCL10 was set at 27.86 and for IFNG at 2.14, i.e. then the donor is classified as infected. The fold change threshold for CXCL10 was set at 4.92 and for IFNG at 1.62, i.e. then the donor is classified as non-infected. For IFNG if the fold change is in the range of 0.7 - 1.1, then the test result is inconclusive and therefore the test is invalid. For CXCL10 if the fold change is in the range of 2.3 - 4.8, then the test result is inconclusive and therefore the test is invalid.

In this experiment the optimized work-flow for the detection of infection with tuberculosis causing pathogens showed high sensitivity and specificity of 88.9 and 94.1% for IFNG, 92.3% and 100% for CXCL10 and 94.4% and 91.2% for IFNG/CXCL10 in combination, respectively. The performance parameters of the test are shown in tab. 9.

Tab. 9: Performance characteristics for the single markers and the combination thereof

|  | IFNG | CXCL10 | IFNG/CXCL10 |
|---|---|---|---|
| **Specificity ( %)** | 94.1 | 100.0 | 91.2 |
| **Sensitivity (%)** | 88.9 | 92.3 | 94.4 |
| **Invalid Rate (%)** | 0.0 | 37.1 | 0.0 |

In case of the combined markers IFNG/CXCL10 the 34 of 36 infected donor were correct classified as shown in table 10.

Tab. 10: Numbers of donors with correct classification

| Donors with correct classification | | | | |
|---|---|---|---|---|
|  | **IFNG** | **CXCL10** | **IFNG/CXCL10** | **Total Donors** |
| **Non infected** | 32 | 18 | 31 | 34 |
| **Infected** | 32 | 24 | 34 | 36 |
| **Invalid** | 0 | 26 | 0 | - |

**Example 4: Stability and integrity of different states of the antigens ESAT-6 and CFP-10 at different storage conditions**

In order to compare the stability and integrity of different states of ESAT6 and CFP-10 proteins at different storage conditions, each 1 mg/ml ESAT-6 (E), CFP-10 (C) and ESAT-6/CFP-10 heterodimer were stored at -20, 4-8 and 25°C.

ESAT-6 (UniProtKB - P9WNK7 (ESXA_MYCTU); AS 1-95) and CFP-10 (UniProtKB - P9WNK5 (ESXB_MYCTU); AS 1-100) from Mycobacterium tuberculosis (strain ATCC 25618 / H37Rv) were produced by Mikrogen GmbH. The heterodimer of *M. tuberculosis* proteins ESAT6 and CFP10 was produced by Lophius Biosciences Gmbh (1 mg/ml ESAT6/CFP-10 complex, K10111; Mikrogen GmbH) (Tab. 1).

The concentration of antigens was analyzed after 18 months of storage at 280 nm by UV-/Vis-spectrophotometry using Thermo Scientific™ NanoDrop™ 1000 UV-/Vis-spectrophotometer.

While the concentration of the ESAT-6/CFP-10 heterodimer (EC) and CFP-10 (C) stayed quite constant at all temperature conditions, the concentration of ESAT-6 (E) decreased at 2-8°C and +25°C vs -20°C, indicating the degradation of ESAT-6 over time at temperatures higher than 0°C.

The integrity of the antigens ESAT-6 and CFP-10 stored as monomer and heterodimer was further analyzed by size exclusion (SE) high performance liquid chromatography (HPLC) using 20 $\mu$l at a Superdex 75 Increase 10/300 (GE Healthcare, now Cytiva) column with 1 x PBS at 0.6 ml/min and detection at 228 nm over 30 min. The runs were performed using a 1260 Infinity System and OpenLAB ChemStation Edition Software (Agilent).

A deviation in the obtained retention time indicates aggregation, i.e. leading to a lower retention time or degradation, i.e. leading to a higher retention time of the respective antigen. While the retention time for CFP-10 and the heterodimer was not significantly altered after 18 months of storage, ESAT-6 stored at +25°C was no longer detectable. The retention times of the heterodimer and also CFP-10 were stable over time, while ESAT-6 was disintegrating over time, particularly at +25°C, with no detectable antigen after 3 months of storage. Slight increase of retention times for ESAT-6 stored at -20 and 4-8°C at month 6 and later indicates partial degradation even at that storage conditions.

The peak width and peak symmetry resulting from the size exclusion chromatography analysis of the antigens ESAT-6, CFP-10 and ESAT-6/CFP-10 heterodimer are also indicating differences in protein integrity. A perfect symmetric peak results in a value of 1. Broader peaks with less symmetry are an indication for aggregation or disintegration resulting in values below 1. Values below 0.5 are resulting from peaks with strong front or tail lines. Increasing values for peak width indicate a loss of integrity, in particular if the peak shows a strong tailing.

The results of peak analysis by analytical size exclusion chromatography (SEC) for ESAT-6, CFP-10 and the heterodimer of ESAT-6 and CFP-10 show that for the heterodimer and the monomeric CFP-10 the peak width stayed constant, whereas for ESAT-6 the peak width increased at storage temperature of 2-8°C indicating a degradation of the ESAT-6 after 18 months of storage. The data for the different time points showed a trend for ESAT-6 monomer to wider peaks at all tested storage temperatures due to more tailing but stayed more or less constant for the ESAT6 /CFP-10 complex and CFP-10.

Summary:

The biochemical analysis of the antigens stored at -20°C, 2-8°C and +25°C showed that

- the initial concentration of the monomeric antigens (1 mg/ml) decreases over time for ESAT-6, but not for CFP-10, indicating the degradation of ESAT-6 as monomer.
- SE HPLC analysis for aggregation and degradation:

  - Retention time stays constant for ESAT6-/CFP-10 heterodimer. ESAT-6 monomer gets lost at 25°C already at time point 3 (6 months).
  - Peak width stays constant for ESAT6-/CFP10 heterodimer with a high level of symmetry. Therefore, the analysis again shows the high stability of the dimeric antigen in direct comparison to the respective monomer, especially ESAT-6.

The obtained data indicate a high stability of ESAT-6-/CFP-10 heterodimer over 18 months storage since all biochemical acceptance criteria were fulfilled.

**Claims**

1. An *in vitro* method of detecting an infection with pathogens causing tuberculosis comprising the steps:

   a) contacting a first aliquot of a sample of an individual with at least one antigen of a pathogen causing tuberculosis, in particular wherein a heterodimer of two antigens, more preferably a heterodimer of ESAT-6 and CFP-10, is added to the first aliquot or wherein the first aliquot is comprised in a container comprising a heterodimer;
   b) incubating the first aliquot with the at least one antigen for about 15 to about 23 hours,
   c) adding a cell lysing reagent which is able to stabilize RNA in a temperature range of about 0°C to about 40°C, more preferably of about 2°C to about 30°C, or of about 15°C to about 30°C, or of about 2°C to about 8°C, or of about 15°C to about 25°C, or about of 18°C to about 30°C for about 2 h to about 48 h, more preferably for about 12 h to about 48 h more preferably for about 2 h to about 72h, more preferably for about 12 h to about 72h,
   d) performing an RNA extraction from the mixture obtained in step c), preferably an automated RNA extraction, wherein RNA is extracted from the mixture obtained in step c) without any prior washing or separation steps;
   e) detecting in the first aliquot and in a second aliquot of the sample of the individual the markers IFNG and CXCL10 and at least one housekeeping gene using a 1-step or 2-step reverse transcription quantitative real-time polymerase chain reaction (RT-qPCR), wherein the second aliquot has not been incubated or stimulated with the at least one antigen, and
   f) comparing all of the detected markers in the first aliquot with the detected markers in the second aliquot, preferably in a fold change analysis, more preferably in a classifier method,

   wherein the sample of the individual in step a) is whole blood, in particular anti-coagulated whole blood, or a purified or isolated PBMC population, lymph or a bronchial lavage and wherein the cell lysing reagent which is able to stabilize RNA comprises a detergens such as such as Triton X 100, Tween 20, Tween 80, 3 [(3 Cholamidopropyl)dimethylammonio] 1 propanesulfonate *(CHAPS),* urea and/or n-Dodecyl-β-D-Maltopyranoside (DDM), a chaotropic agent such as a guanidinium lysis buffers comprising preferably guanidinium isothiocyanate and/or guanidinium-HCl and an antioxidant such as DTT, Dithioerythritol (DTE), Tris(2-carboxyethyl)phosphin (TCEP) and/or β-Mercaptoethanolopic agent.

2. The method according to any one of the preceding claims, wherein the mixture obtained in step c) is mixed.

3. The method according to any one of the preceding claims, wherein the automated RNA extraction in step d) is performed in a solid phase extraction method using silica beads, in particular magnetic silica beads, for absorbing RNA.

4. The method according to any one of the preceding claims, wherein the at least one housekeeping gene in step e) is RPLPO.

5. The method according to any one of the preceding claims, wherein IFNG and RPLPO are detected in step e).

6. The method according to any one of the preceding claims, wherein IFNG, CXCL10 and RPLPO are detected in step e).

7. The method according to any one of the preceding claims, wherein the RT-qPCR is performed by using at least two, more preferably at least three, dyes, in particular wherein at least one dye is FAM, HEX or Cy5.

8. The method according to any one of the preceding claims, wherein step a) comprises additionally a step of contacting a third aliquot of a sample of an individual with a stimulation control, wherein the stimulation control stimulates cells to produce IFNG, in particular wherein the stimulation control is Phytohemagglutinin (PHA), Lipopolysaccharide (LPS), anti-CD3/anti-CD28, Staphylococcal enterotoxin B (SEB), PMA (Phorbol 12-myristate 13-acetate)/Ionomycin.

9. The method according to any one of the preceding claims, wherein step e) is additionally performed for a positive control, wherein the positive control is a plasmid comprising fragments of the marker RNAs and the housekeeping gene(s) to be detected in step e), wherein said fragments comprise at least the nucleic acid sequences of the marker RNAs and the housekeeping gene(s) to which the corresponding primers and probes hybridize or align.

10. The method according to any one of the preceding claims, wherein steps d) and e) are additionally performed for a negative extraction control, wherein the negative extraction control comprises a mixture of PBS and the cell lysing reagent which is able to stabilize RNA as used in step c), preferably a mixture of 1:1.25.

11. The method according to any one of the preceding claims, wherein the method comprises additionally a step of adding an inhibition control to the first, second and/or third aliquot and detecting said inhibition control in step e).

12. A kit for performing the method according to any one of the preceding claims or one or more single steps thereof, the kit comprising:

  - a heterodimer of ESAT-6 and CFP-10,
  - a stimulation control, wherein the stimulation control stimulates cells to produce IFNG and
  - a cell lysing reagent which is able to stabilize RNA, wherein the cell lysing reagent is preferably provided in a light-protected packaging (e.g. sleeve).

13. A kit for performing the method according to any one of claims 1 to 11 or one or more single steps thereof, the kit comprising:

  - a reaction mixture comprising desoxynucleotides (dATP, dGTP, dCTP, dTTP) and a polymerase buffer solution,
  - a mixture comprising primer and probes for detecting IFNG, CXCL10, and at least one housekeeping gene, wherein each probe comprises preferably a dye and a quencher,
  - a Reverse Transcriptase,
  - a positive control, wherein the positive control is a plasmid comprising fragments of the marker RNAs and the housekeeping gene(s) to be detected in step e), wherein said fragments comprise at least the nucleic acid sequences of the marker RNAs and the housekeeping gene(s) to which the corresponding primers and probes hybridize or align, and

a negative extraction control, wherein the negative extraction control comprises a mixture of the cell lysing reagent which is able to stabilize RNA as used in step c) of the method according to any one of claims 1 to 11 and PBS, wherein the cell lysing reagent which is able to stabilize RNA comprises a detergens such as such as Triton X 100, Tween 20, Tween 80, 3 [(3 Cholamidopropyl)dimethylammonio] 1 propanesulfonate *(CHAPS),* urea and/or n-Dodecyl-β-D-Maltopyranoside (DDM), a chaotropic agent such as a guanidinium lysis buffers comprising preferably guanidinium isothiocyanate and/or guanidinium-HCl and an antioxidant such as DTT, Dithioerythritol (DTE), Tris(2-carboxyethyl) phosphin (TCEP) and/or β-Mercaptoethanolopic agent.

**Patentansprüche**

1. Ein in-vitro-Verfahren zum Nachweis einer Infektion mit Tuberkulose verursachenden Krankheitserregern, das die folgenden Schritte umfasst:

   a) Inkontaktbringen eines ersten Aliquots einer Probe eines Individuums mit mindestens einem Antigen eines Tuberkulose verursachenden Krankheitserregers, wobei insbesondere ein Heterodimer aus zwei Antigenen, vorzugsweise ein Heterodimer aus ESAT-6 und CFP-10, zu dem ersten Aliquot hinzugefügt wird oder wobei das erste Aliquot in einem Behälter enthalten ist, der ein Heterodimer umfasst;
   b) Inkubieren des ersten Aliquots mit dem mindestens einen Antigen für etwa 15 bis etwa 23 Stunden,
   c) Zugabe eines zelllysierenden Reagenzes, das in der Lage ist, RNA in einem Temperaturbereich von etwa 0°C bis etwa 40°C, bevorzugter von etwa 2°C bis etwa 30°C oder von etwa 15°C bis etwa 30°C oder von etwa 2°C bis etwa 8°C oder von etwa 15°C bis etwa 25°C oder von etwa 18°C bis etwa 30°C für etwa 2 h bis etwa 48 h, bevorzugter für etwa 12 h bis etwa 48 h, bevorzugter für etwa 2 h bis etwa 72 h, bevorzugter für etwa 12 h bis etwa 72 h, zu stabilisieren,
   d) Durchführen einer RNA-Extraktion aus der in Schritt c) erhaltenen Mischung, vorzugsweise einer automatisierten RNA-Extraktion, wobei die RNA aus der in Schritt c) erhaltenen Mischung ohne vorherige Wasch- oder Trennschritte extrahiert wird;
   e) Nachweisen der Marker IFNG und CXCL10 und mindestens eines Housekeeping-Gens in dem ersten Aliquot und in einem zweiten Aliquot der Probe des Individuums unter Verwendung einer 1 Schritt oder 2 Schritt quantitativen Echtzeit- Reverse Transkriptase-Polymerase-Kettenreaktion (RT-qPCR), wobei das zweite Aliquot nicht mit dem mindestens einen Antigen inkubiert oder stimuliert worden ist, und
   f) Vergleich aller nachgewiesenen Marker im ersten Aliquot mit den nachgewiesenen Markern im zweiten Aliquot, vorzugsweise in einer Fold-Change-Analyse, noch bevorzugter in einem Klassifizierungsverfahren,
   wobei die Probe des Individuums in Schritt a) Vollblut, insbesondere antikoaguliertes Vollblut, oder eine gereinigte oder isolierte PBMC-Population, Lymphe oder eine Bronchiallavage ist und
   wobei das zelllysierende Reagenz, das in der Lage ist, RNA zu stabilisieren, ein Detergens, wie z.B. Triton X 100, Tween 20, Tween 80, 3 [(3 Cholamidopropyl)dimethylammonio] 1 propansulfonat (CHAPS), Harnstoff und/oder n-Dodecyl-β-D-Maltopyranosid (DDM), ein chaotropes Mittel wie einen Guanidinium-Lysepuffer umfassend vorzugsweise Guanidinium-Isothiocyanat und/oder Guanidinium-HCl, und ein Antioxidationsmittel wie DTT, Dithioerythritol (DTE), Tris(2-carboxyethyl)phosphin (TCEP) und/oder β- mercaptoethanolopisches Mittel umfasst.

2. Das Verfahren nach einem der vorhergehenden Ansprüche, wobei die in Schritt c) erhaltene Mischung gemischt wird.

3. Das Verfahren nach einem der vorhergehenden Ansprüche, wobei die automatisierte RNA-Extraktion in Schritt d) in einem Festphasenextraktionsverfahren unter Verwendung von Siliziumdioxidbeads, insbesondere magnetischen Siliziumdioxidbeads, zur Absorption von RNA durchgeführt wird.

4. Das Verfahren nach einem der vorhergehenden Ansprüche, wobei das mindestens eine Housekeeping-Gen in Schritt e) RPLPO ist.

5. Das Verfahren nach einem der vorhergehenden Ansprüche, wobei IFNG und RPLPO in Schritt e) nachgewiesen werden.

6. Das Verfahren nach einem der vorhergehenden Ansprüche, wobei IFNG, CXCL10 und RPLPO in Schritt e) nachgewiesen werden.

7. Das Verfahren nach einem der vorhergehenden Ansprüche, wobei die RT-qPCT durchgeführt wird unter Verwendung von mindestens zwei, bevorzugter mindestens drei Farbstoffen, wobei insbesondere mindestens ein Farbstoff FAM, HEX oder Cy5 ist.

8. Das Verfahren nach einem der vorhergehenden Ansprüche, wobei Schritt a) zusätzlich einen Schritt des Inkontaktbringens eines dritten Aliquots einer Probe eines Individuums mit einer Stimulationskontrolle umfasst, wobei die Stimulationskontrolle Zellen zur Produktion von IFNG stimuliert, wobei es sich bei der Stimulationskontrolle insbesondere um Phytohämagglutinin (PHA), Lipopolysaccharid (LPS), Anti-CD3/Anti-CD28, Staphylokokken-Enterotoxin B (SEB), PMA (Phorbol 12-Myristat 13-Acetat)/Ionomycin handelt.

9. Das Verfahren nach einem der vorhergehenden Ansprüche, wobei Schritt e) zusätzlich für eine Positivkontrolle durchgeführt wird, wobei die Positivkontrolle ein Plasmid ist, das Fragmente der Marker-RNAs und des/der Housekeeping-Gens/e umfasst, die in Schritt e) nachgewiesen werden sollen, wobei die Fragmente zumindest die Nukleinsäuresequenzen der Marker-RNAs und des/der Housekeeping-Gens/e umfassen, an die die entsprechenden Primer und Sonden hybridisieren oder sich ausrichten.

10. Das Verfahren nach einem der vorhergehenden Ansprüche, wobei die Schritte d) und e) zusätzlich für eine negative Extraktionskontrolle durchgeführt werden, wobei die negative Extraktionskontrolle ein Gemisch aus PBS und dem zelllysierenden Reagenz, das in der Lage ist, RNA zu stabilisieren, wie in Schritt c) verwendet, umfasst, vorzugsweise ein Gemisch von 1:1,25.

11. Das Verfahren nach einem der vorhergehenden Ansprüche, wobei das Verfahren zusätzlich einen Schritt der Zugabe einer Inhibitionskontrolle zu dem ersten, zweiten und/oder dritten Aliquot und den Nachweis dieser Inhibitionskontrolle in Schritt e) umfasst.

12. Ein Kit zur Durchführung des Verfahrens nach einem der vorhergehenden Ansprüche oder eines oder mehrerer Einzelschritte davon, wobei der Kit umfasst:

   - ein Heterodimer aus ESAT-6 und CFP-10,
   - eine Stimulationskontrolle, wobei die Stimulationskontrolle die Zellen zur Produktion von IFNG stimuliert, und
   - ein zelllysierendes Reagenz, das in der Lage ist, RNA zu stabilisieren, wobei das zelllysierende Reagenz vorzugsweise in einer lichtgeschützten Verpackung (z.B. Hülle) bereitgestellt wird.

13. Ein Kit zur Durchführung des Verfahrens nach einem der Ansprüche 1 bis 11 oder eines oder mehrerer Einzelschritte davon, wobei das Kit umfasst:

   - eine Reaktionsmischung umfassend die Desoxynukleotide (dATP, dGTP, dCTP, dTTP) und eine Polymerasepufferlösung,
   - ein Gemisch umfassend Primer und Sonden zum Nachweis von IFNG, CXCL10 und mindestens einem Housekeeping-Gen, wobei jede Sonde vorzugsweise einen Farbstoff und einen Quencher umfasst,
   - eine Reverse Transkriptase,
   - eine Positivkontrolle, wobei die Positivkontrolle ein Plasmid ist, das Fragmente der Marker-RNAs und des/der in Schritt e) nachzuweisenden Housekeeping-Gens/e umfasst, wobei die Fragmente zumindest die Nukleinsäuresequenzen der Marker-RNAs und des/der Housekeeping-Gens/e umfassen, an die die entsprechenden Primer und Sonden hybridisieren oder sich ausrichten, und

   eine negative Extraktionskontrolle, wobei die negative Extraktionskontrolle ein Gemisch aus dem zelllysierenden Reagenz, das in der Lage ist, RNA zu stabilisieren, wie es in Schritt c) des Verfahrens nach einem der Ansprüche 1 bis 11 verwendet wird, und PBS umfasst, wobei das zelllysierende Reagenz, das in der Lage ist, RNA zu stabilisieren, ein Detergens wie beispielsweise Triton X 100, Tween 20, Tween 80, 3 [(3 Cholamidopropyl)dimethylammonio] 1 propansulfonat (CHAPS), Harnstoff und/oder n-Dodecyl-$\beta$-D-Maltopyranosid (DDM), ein chaotropes Mittel wie einen Guanidinium-Lysepuffer vorzugsweise umfassend Guanidinium-Isothiocyanat und/oder Guanidinium-HCl, und ein Antioxidationsmittel wie DTT, Dithioerythritol (DTE), Tris(2-carboxyethyl)phosphin (TCEP) und/oder $\beta$-mercaptoethanolopisches Mittel umfasst.

**Revendications**

1. Méthode *in vitro* de détection d'une infection par des pathogènes provoquant la tuberculose, comprenant les étapes de :

a) mise en contact d'une première aliquote d'un échantillon d'un individu avec au moins un antigène d'un pathogène provoquant la tuberculose, en particulier dans laquelle un hétérodimère de deux antigènes, mieux encore un hétérodimère d'ESAT-6 et de CFP-10, est ajouté à la première aliquote, ou dans laquelle la première aliquote est comprise dans un récipient comprenant un hétérodimère ;

b) incubation de la première aliquote avec l'au moins un antigène pendant environ 15 à environ 23 heures ;

c) ajout d'un réactif de lyse cellulaire qui est de capable de stabiliser l'ARN dans une plage de température allant d'environ 0 °C à environ 40 °C, mieux encore d'environ 2 °C à environ 30 °C, ou d'environ 15 °C à environ 30 °C, ou d'environ 2 °C à environ 8 °C, ou d'environ 15 °C à environ 25 °C, ou d'environ 18 °C à environ 30 °C, pendant environ 2 heures à environ 48 heures, mieux encore pendant environ 12 heures à environ 48 heures, mieux encore pendant environ 2 heures à environ 72 heures, mieux encore pendant environ 12 heures à environ 72 heures ;

d) mise en œuvre d'une extraction d'ARN à partir du mélange obtenu dans l'étape c), de préférence d'une extraction d'ARN automatisée, dans laquelle de l'ARN est extrait à partir du mélange obtenu dans l'étape c) sans aucune étape préalable de lavage ou de séparation ;

e) détection, dans la première aliquote et dans une deuxième aliquote de l'échantillon de l'individu, des marqueurs IFNG et CXCL10 et d'au moins un gène domestique, utilisant une amplification en chaîne par polymérase en temps réel quantitative après transcription inverse (RT-qPCR) en 1 étape ou en 2 étapes, dans laquelle la deuxième aliquote n'a pas été incubée ou stimulée avec l'au moins un antigène, et

f) comparaison de tous les marqueurs détectés dans la première aliquote avec les marqueurs détectés dans la deuxième aliquote, de préférence dans une analyse de variation de facteur, mieux encore dans une méthode de classification,

dans laquelle l'échantillon de l'individu dans l'étape a) est le sang entier, en particulier du sang entier anticoagulé, ou une population de PBMC purifiées ou isolées, de la lymphe, ou un lavage bronchique, et

dans laquelle le réactif de lyse cellulaire qui est capable de stabiliser l'ARN comprend un détergent tel que le Triton X 100, le Tween 20, le Tween 80, le 3-[(3-cholamidopropyl)diméthylammonio]-1-propanesulfonate (*CHAPS*), l'urée et/ou le n-dodécyl-β-D-maltopyranoside (DMM), un agent chaotrope tel que les tampons de lyse au guanidinium comprenant de préférence de l'isothiocyanate de guanidinium et/ou du guanidinium-HCl et un antioxydant tel que le DTT, le dithioérythritol (DTE), la tris(2-carboxyéthyl)phosphine (TCEP) et/ou un agent β-mercaptoéthanolique.

2. Méthode selon la revendication précédente, dans laquelle le mélange obtenu dans l'étape c) est mélangé.

3. Méthode selon l'une quelconque des revendications précédentes, dans laquelle l'extraction d'ARN automatisée dans l'étape d) est effectuée dans une méthode d'extraction en phase solide utilisant des billes de silice, en particulier des billes de silice magnétiques, pour absorber l'ARN.

4. Méthode selon l'une quelconque des revendications précédentes, dans laquelle l'au moins un gène domestique dans l'étape e) est RPLPO.

5. Méthode selon l'une quelconque des revendications précédentes, dans laquelle les IFNG et RPLPO sont détectés dans l'étape e).

6. Méthode selon l'une quelconque des revendications précédentes, dans laquelle les IFNG, CXCL10 et RPLPO sont détectés dans l'étape e).

7. Méthode selon l'une quelconque des revendications précédentes, dans laquelle la RT-qPCR est effectuée par utilisation d'au moins deux, mieux encore d'au moins trois colorants, en particulier dans laquelle au moins un colorant est FAM, HEX ou Cy5.

8. Méthode selon l'une quelconque des revendications précédentes, dans laquelle l'étape a) comprend de plus une étape de mise en contact d'une troisième aliquote d'un échantillon d'un individu avec un témoin de stimulation, dans laquelle le témoin de stimulation stimule les cellules pour qu'elles produisent de l'IFNG, en particulier dans laquelle le témoin de stimulation est une phytohémagglutinine (HPA), un lipopolysaccharide (LPS), un anti-CD3/anti-CD28, une entérotoxine staphylococcique B (SEB), le PMA (12-myristate-13-acétate de phorbol)/ionomycine.

9. Méthode selon l'une quelconque des revendications précédentes, dans laquelle l'étape e) est de plus effectuée pour un témoin positif, dans laquelle le témoin positif est un plasmide comprenant des fragments des ARN marqueurs et du ou gènes domestiques devant être détectés dans l'étape e), dans laquelle lesdits fragments comprennent au moins les séquences d'acide nucléique des ARN marqueurs et du ou des gènes domestiques avec lesquelles les sondes et amorces correspondantes s'hybrident ou s'alignent.

10. Méthode selon l'une quelconque des revendications précédentes, dans laquelle les étapes d) et e) sont de plus effectuées pour un témoin d'extraction négatif, dans laquelle le témoin d'extraction négatif comprend un mélange de STP et du réactif de lyse cellulaire qui est capable de stabiliser l'ARN tel qu'utilisé dans l'étape c), de préférence un mélange 1/1,25.

11. Méthode selon l'une quelconque des revendications précédentes, laquelle méthode comprend de plus une étape d'addition d'un témoin d'inhibition aux première, deuxième et/ou troisième aliquotes, et de détection dudit témoin d'inhibition dans l'étape e).

12. Trousse pour mettre en œuvre le procédé selon l'une quelconque des revendications précédentes ou une ou plusieurs étapes isolées de celui-ci, la trousse comprenant :

- un hétérodimère d'ESAT-6 et de CFP-10,
- un témoin de stimulation, lequel témoin de stimulation stimule des cellules pour qu'elles produisent de l'IFNG, et
- un réactif de lyse cellulaire qui est capable de stabiliser l'ARN, lequel réactif de lyse cellulaire est de préférence fourni dans un conditionnement à l'abri de la lumière (par exemple une pochette).

13. Trousse pour mettre en œuvre le procédé selon l'une quelconque des revendications 11 ou une ou plusieurs étapes isolées de celui-ci, la trousse comprenant :

- un mélange réactionnel comprenant des désoxynucléotides (dATP, dGTP, dCTP, dTTP) et une solution tampon de polymérase,
- un mélange comprenant une amorce et des sondes pour détecter l'IFNG, la CXCL10 et au moins un gène domestique, dans lequel chaque sonde comprend de préférence un colorant et un extincteur,
- une transcriptase inverse,
- un témoin positif, lequel témoin positif est un plasmide comprenant des fragments des ARN marqueurs et du ou des gènes domestiques devant être détectés dans l'étape e), dans lequel lesdits fragments comprennent au moins les séquences d'acide nucléique des ARN marqueurs et du ou des gènes domestiques avec lesquelles les sondes et amorces correspondantes s'hybrident ou s'alignent, et
- un témoin d'extraction négatif, lequel témoin d'extraction négatif comprend un mélange du réactif de lyse cellulaire qui est capable de stabiliser l'ARN tel qu'utilisé dans l'étape c) de la méthode selon l'une quelconque des revendications 1 à 11 et de la STP, dans lequel le réactif de lyse cellulaire qui est capable de stabiliser l'ARN comprend un détergent tel que le Triton X 100, le Tween 20, le Tween 80, le 3-[(3-cholamidopropyl)diméthylammonio]-1-propanesulfonate (*CHAPS*), l'urée et/ou le n-dodécyl-β-D-maltopyranoside (DMM), un agent chaotrope tel que les tampons de lyse au guanidinium comprenant de préférence de l'isothiocyanate de guanidinium et/ou du guanidinium-HCl et un antioxydant tel que le DTT, le dithioérythritol (DTE), la tris(2-carboxyéthyl)phosphine (TCEP) et/ou un agent β-mercaptoéthanolique.

Fig. 1

Fig. 2A

Fig. 2B

Fig. 3A

Fig. 3B

Fig. 4A

Fig. 4B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008028489 A3

- WO 2012037937 A2

### Non-patent literature cited in the description

- **DOAN et al.** *PLOS ONE*, 2018, vol. 12 (11), e0188631
- **SESTER et al.** *Eur. Resp. J.*, 2011, vol. 37, 100
- Tuberculosis IGRA TB Test Policy Statement. *World Health Organization*, 2011
- **BLAUENFELD et al.** *PLOS ONE*, 2014, vol. 9, e105628
- **KIM et al.** *J. Mol. Diagn.*, 2015, vol. 17 (1), 90-99
- *CHEMICAL ABSTRACTS*, 91809-66-4
- **REICHELT et al.** *Protein Expression and Purification*, 2006, vol. 46, 483-488
- **BAKER et al.** *Nature Methods*, October 2005, vol. 2 (10), 731-734
- **BREIMAN**. Random Forests. *Machine Learning.*, 2001, vol. 45, 5-32
- **LEO BREIMAN**. *Machine Learning.*, 2001, vol. 45 (1), 5-32

- **MALLEY et al.** *Methods Inf Med*, 2012, vol. 51, 74-81, http://dx.doi.org/10.3414/ME00-01-0052
- **GEURTS et al.** *Machine Learning.*, 2006, vol. 63, 3-42
- **LEWINSOHN et al.** Official American Thoracic Society/Infectious Diseases Society of America/Centers for Disease Control and Prevention Clinical Practice Guidelines: Diagnosis of Tuberculosis in Adults and Children. *CID*, 2016, vol. 00 (0), 1-33
- **NAHID et al.** Official American Thoracic Society/-Centers for Disease Control and Prevention/Infectious Diseases Society of America Clinical Practice Guidelines: Treatment of Drug-Susceptible Tuberculosis. *ATS/TS/CDC/IDSA Clinical Practice Guidelines for Drug-Susceptible TB • CID*, 01 October 2016, vol. 63, e147-e195